(19) Europäisches Patentamt
European Patent Office
Office européen des brevets

(11) EP 0 661 258 B1

(12) FASCICULE DE BREVET EUROPEEN

(45) Date de publication et mention de la délivrance du brevet: **08.04.1998 Bulletin 1998/15**

(21) Numéro de dépôt: **94402552.7**

(22) Date de dépôt: **10.11.1994**

(51) Int. Cl.[6]: **C07C 65/19**, C07C 65/38, C07C 65/40, C07C 313/04, C07C 317/22, C07C 235/46, C07C 65/28, C07C 69/88, C07C 69/78, C07C 69/90, C07C 69/92, C07D 335/06, C07D 333/38, C07D 207/40

(54) **Nouveaux composés propynyl bi-aromatiques, compositions pharmaceutiques et cosmétiques les contenant et utilisations**

Biaromatische Propinylverbindungen, sie enthaltende pharmazeutische Zubereitungen und Kosmetika sowie deren Verwendungen

Biaromatic propynyl compounds, pharmaceutical compositions and cosmetics containing them and their uses

(84) Etats contractants désignés:
**AT BE CH DE DK ES FR GB GR IE IT LI LU MC NL PT SE**

(30) Priorité: **15.12.1993 FR 9315067**

(43) Date de publication de la demande: **05.07.1995 Bulletin 1995/27**

(73) Titulaire:
**CENTRE INTERNATIONAL DE RECHERCHES DERMATOLOGIQUES GALDERMA, ( CIRD GALDERMA)**
**06560 Valbonne (FR)**

(72) Inventeur:
**Bernardon, Jean-Michel**
**F-06650 Le Rouret (FR)**

(74) Mandataire:
**Tezier Herman, Béatrice**
**L'OREAL,**
**Département Propriété Industrielle,**
**90, rue du Gal Roguet**
**92583 Clichy Cédex (FR)**

(56) Documents cités:
**EP-A- 0 170 105**
**EP-A- 0 401 517**
**EP-A- 0 476 645**
**EP-A- 0 476 658**
**EP-A- 0 514 264**
**WO-A-92/20643**

- **Cancer Research 43, 5268-5272 (1983)**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 661 258 B1

Printed by Xerox (UK) Business Services
2.15.12/3.4

## Description

L'invention concerne, à titre de produits industriels nouveaux et utiles, des composés bi-aromatiques présentant un motif propynyl. Elle concerne également l'utilisation de ces nouveaux composés dans des compositions pharmaceutiques destinées à un usage en médecine humaine ou vétérinaire, ou bien encore dans des compositions cosmétiques.

Des composés ont été décrits dans les demandes de brevets EP-A-0401517, EP-A-0476645 et EP-A-0476658. Ces composés présentent des activités inhibitrices de l'entrée du calcium dans les leucocytes et/ou thrombocytes ou inhibitrices de la cyclooxygénase et/ou de la 5-lipooxygénase.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante :

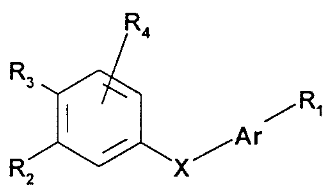

(I)

dans laquelle :

* Ar représente un radical choisi parmi les radicaux de formules (a)-(e) suivantes :

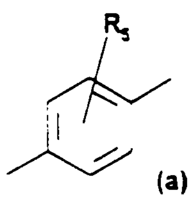
(a)

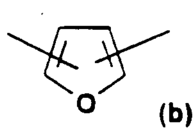
(b)

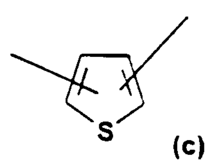
(c)

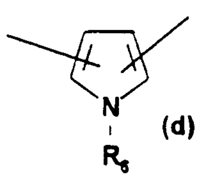
(d)

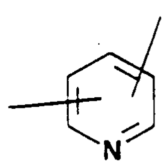
(e)

$R_5$ et $R_6$ ayant la signification donnée ci-après,

* $R_1$ représente :

(i) un atome d'hydrogène
(ii) un radical $-CH_3$
(iii) un radical $-CH_2-O-R_6$
(iv) un radical $-O-R_6$
(v) un radical $-CO-R_7$,
(vi) un radical $-S(O)_tR_9$

$R_6$, $R_7$, $R_9$ et t ayant la signification donnée ci-après,

- * X représente un radical de formule :

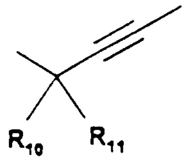

ou

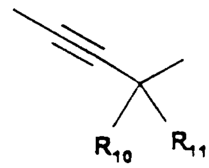

$R_{10}$ et $R_{11}$ ayant la signification donnée ci-après,

- * $R_2$ et $R_3$ représentent un atome d'hydrogène, un radical alkyle linéaire ou ramifié ayant de 1 à 20 atomes de carbone, un radical $-OR_6$ ou un radical $-SR_6$, $R_6$ ayant la signification donnée ci-après, étant entendu que $R_2$ et $R_3$, pris ensemble, peuvent former avec le cycle aromatique adjacent un cycle à 5 ou 6 chainons éventuellement substitué par des groupes méthyle et/ou éventuellement interrompu par un atome d'oxygène ou de soufre.
- * $R_4$ représente un atome d'hydrogène, un atome d'halogène, un radical alkyle inférieur ou un radical $-OR_6$, $R_6$ ayant la signification donnée ci-après,

étant entendu que dans tout ce qui précède :

- $R_5$ a la même signification que $R_4$,
- $R_6$ représente un atome d'hydrogène, un radical alkyle inférieur ou un radical $-CO-R_9$, $R_9$ ayant la signification donnée ci-après,
- $R_7$ représente :

  (a) un atome d'hydrogène
  (b) un radical alkyle inférieur
  (c) un radical de formule :

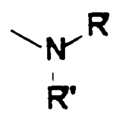

  R' et R'' ayant la signification donnée ci-après,
  (d) un radical $-OR_8$, $R_8$ ayant la signification donnée ci-après,

- $R_8$ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- $R_9$ représente un radical alkyle inférieur,
- $R_{10}$ représente un atome d'hydrogène, un radical alkyle inférieur ou un radical $-OR_6$,
- $R_{11}$ représente un radical $-OR_6$,
- R' et R'' représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- t est un nombre entier égal à 0,1 ou 2,
- les radicaux $R_{10}$ et $R_{11}$ ci-dessus, pris ensemble, peuvent former un radical oxo unique de formule =O,

avec les conditions suivantes :

- si R10 représente un atome d'hydrogène, R11 un radical hydroxyle, et R2 et R3 ne forment pas avec le cycle aromatique adjacent un cycle à 5 ou 6 chainons, alors R4 ne représente pas un radical hydroxyle, quand R4 est en position ortho de la position $\alpha$ du cycle ;
- si R10 représente un atome d'hydrogène et R11 un radical hydroxyle, ou R10 et R11 forment un radical oxo unique de formule =O, et R2 et R3 ne forment pas avec le cycle aromatique adjacent un cycle à 5 ou 6 chainons, alors R5

ne représente pas un radical hydroxyle, quand R5 est en position ortho de la position $\alpha$ du cycle Ar de formule (a);

- si R10 représente un atome d'hydrogène et R11 un radical hydroxyle, ou R10 et R11 forment un radical oxo unique de formule =O, R2 et R3 ne forment pas avec le cycle aromatique adjacent un cycle à 5 ou 6 chainons et Ar représente un radical de formula (a), (b), (c) ou (e), alors R1 ne représente pas (i), (ii) ou (iv), quand R6 représente un radical alkyle en C1-C6.

L'invention vise également les sels des composés de formule (I) ci-dessus dans le cas où $R_1$ représente une fonction acide carboxylique, ainsi que les isomères optiques et géométriques desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle, et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 1 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en $C_1$-$C_6$ ou mono ou polyhydroxyalkyle tels que définis ci-dessus.

Lorsque $R_4$ et $R_5$ représente un atome d'halogène, celui-ci est de préférence un atome de fluor, de chlore et de brome.

Parmi les composés de formule (I) ci-dessus rentrant dans le cadre de la présente invention, on peut notamment citer les suivants :

- 4-[3-oxo-3-(5,6,7,8-tétrahydro-5,5,8,8-tétraméthyl-2-naphtyl)-1-propynyl]benzoate de méthyle
- 4-[3-hydroxy-3-(5,6,7,8-tétrahydro-5,5,8,8-tétraméthyl-2-naphtyl)-1-propynyl]benzoate de méthyle
- Acide 4-[3-hydroxy-3-(5,6,7,8-tétrahydro-5,5,8,8-tétraméthyl-2-naphtyl)-1-propynyl]benzoïque
- Acide 4-[3-oxo-3-(5,6,7,8-tétrahydro-5,5,8,8-tétraméthyl-2-naphtyl)-1-propynyl]benzoïque
- Acide 2-hydroxy-4-[3-oxo-3-(5,6,7,8-tétrahydro(5,5,8,8-tétraméthyl-2-naphtyl)-1-propynyl]benzoïque
- 2-hydroxy-4-[3-hydroxy-3-(5,6,7,8-tétrahydro-5,5,8,8-tétraméthyl-2-naphtyl)-1-propynyl]benzoate de méthyle
- Acide 2-hydroxy-4-[3-hydroxy-3-(5,6,7,8-tétrahydro-5,5,8,8-tétraméthyl-2-naphtyl)-1-propynyl]benzoïque
- Acide 2-hydroxy-4-[3-oxo-3-(3-tert-butyl-4-méthoxyphényl)-1-propynyl]benzoïque
- Acide 4-[1-oxo-3-(5,6,7,8-tétrahydro-5,5,8,8-tétraméthyl-2-naphtyl)-2-propynyl]benzoïque
- Acide 4-[1-hydroxy-3-(5,6,7,8-tétrahydro-5,5,8,8-tétraméthyl-2-naphtyl)-2-propynyl]benzoïque
- 2-hydroxy-4-[3-hydroxy-3-(4,4-diméthylthiochroman-6-yl)-1-propynyl]benzoate de méthyle
- Acide 2-hydroxy-4-[3-oxo-3-(4,4-diméthylthiochroman-6-yl)-1-propynyl]benzoïque
- Acide 4-[3-hydroxy-3-(3-tert-butyl-4-méthoxyphényl)-1-propynyl]benzoïque
- N-méthyl-4-[3-hydroxy-3-(5,6,7,8-tétrahydro-5,5,8,8-tétraméthyl-2-naphtyl)-1-propynyl]-2-pyrrole carboxylate de méthyle
- Acide N-méthyl-4-[3-hydroxy-3-(5,6,7,8-tétrahydro-5,5,8,8-tétraméthyl-2-naphtyl)-1-propynyl]-2-pyrrole carboxylique
- 4-[3-hydroxy-3-(5,6,7,8-tétrahydro-5,5,8,8-tétraméthyl-2-naphtyl)-1-propynyl]-2-pyrrole carboxylate de méthyle
- 2-hydroxy-4-[3-hydroxy-3-(4,4-diméthylthiochromam-7-yl)-1-propynyl]benzoate de méthyle
- Acide 4-[1-hydroxy-3-(4,4-diméthylthiochroman-6-yl)-2-propynyl]benzoïque

- Acide 2-hydroxy-4-[3-hydroxy-3-(4,4-diméthylthiochroman-6-yl)-1-propynyl]benzoïque
- Acide 2-hydroxy-4-[3-hydroxy-3-(4,4-diméthylthiochroman-7-yl)-1-propynyl]benzoïque
- Isomère (+) du 4-[3-hydroxy-3-(5,6,7,8-tétrahydro-5,5,8,8-tétraméthyl-2-naphtyl)-1-propynyl]benzoate de méthyle
- Isomère (-) de l'acide 4-(3-hydroxy-3-(5,6,7,8-tétrahydro-5,5,8,8-tétraméthyl-2-naphtyl)-1-propynyl]benzoïque
- Isomère (+) du 2-hydroxy-4-[3-hydroxy-3-(5,6,7,8-tétrahydro-5,5,8,8-tétraméthyl-2-naphtyl)-1-propynyl]benzoate de méthyle
- Isomère (-) de l'acide 2-hydroxy-4-[3-hydroxy-3-(5,6,7,8-tétrahydro-5,5,8,8-tétramethyl-2-naphtyl)-1-propynyl]benzoïque
- Isomère (-) du 4-[3-hydroxy-3-(5,6,7,8-tétrahydro-5,5,8,8-tétraméthyl-2-naphtyl)-1-propynyl]benzoate de méthyle
- Isomère (-) du 2-hydroxy-4-[3-hydroxy-3-(5,6,7,8-tétrahydro-5,5,8,8-tétraméthyl-2-naphtyl)-1-propynyl]benzoate de méthyle
- Isomère (+) de l'acide 4-[3-hydroxy-3-(5,6,7,8-tétrahydro-5,5,8,8-tétraméthyl-2-naphtyl)-1-propynyl]benzoïque
- Isomère (+) de l'acide 2-hydroxy-4-[3-hydroxy-3-(5,6,7,8-tétrahydro-5,5,8,8-tétraméthyl-2-naphtyl)-1-propynyl]benzoïque
- 2-hydroxy-4-[3-hydroxy-3-méthyl-3-(5,6,7,8-tétrahydro-5,5,8,8-tétraméthyl-2-naphtyl)-1-propynyl]benzoate de méthyle
- Acide 2-hydroxy-4-[3-hydroxy-3-méthyl-3-(5,6,7,8-tétrahydro-5,5,8,8-tétraméthyl-2-naphtyl)-1-propynyl]benzoïque
- Acide 2-méthoxy-4-[3-hydroxy-3-(5,6,7,8-tétrahydro-5,5,8,8-tétraméthyl-2-naphtyl)-1-propynyl]benzoïque
- 4-[3-hydroxy-3-(5,6,7,8-tétrahydro-5,5,8,8-tétraméthyl-2-naphtyl)-1-propynyl]benzaldéhyde
- Acétate de 4-[3-hydroxy-3-(5,6,7,8-tétrahydro-5,5,8,8-tétraméthyl-2-naphtyl)-1-propynyl]benzyle
- 4-[3-hydroxy-3-(5,6,7,8-tétrahydro-5,5,8,8-tétraméthyl-2-naphtyl)-1-propynyl]benzeneméthanol
- 4-[3-hydroxy-3-(5,6,7,8-tétrahydro-5,5,8,8-tétraméthyl-2-naphtyl)-1-propynyl]toluène
- Acetate de 4-[3-hydroxy-3-(5,6,7,8-tétrahydro-3,5,5,8,8-pentaméthyl-2-naphtyl)-1-propynyl]phényle
- 4-[3-hydroxy-3-(5,6,7,8-tétrahydro-3,5,5,8,8-pentaméthyl-2-naphtyl)-1-propynyl]phénol
- 4-[3-hydroxy-3-(5,6,7,8-tétrahydro-5,5,8,8-tétraméthyl-2-naphtyl)-1-propynyl]phénylsulfinylméthane
- 4-[3-hydroxy-3-(5,6,7,8-tétrahydro-5,5,8,8-tétraméthyl-2-naphtyl)-1-propynyl] phénylsulfonylméthane
- N-éthyl-4-[3-hydroxy-3-(5,6,7,8-tétrahydro-5,5,8,8-tétraméthyl-2-naphtyl)-1-propynyl]benzamide
- N,N'-diéthyl-4-[3-hydroxy-3-(5,6,7,8-tétrahydro-5,5,8,8-tétraméthyl-2-naphtyl)-1-propynyl]benzamide
- Morpholide de l'acide 4-[3-hydroxy-3-(5,6,7,8-tétrahydro-5,5,8,8-tétraméthyl-2-naphtyl)-1-propynyl]benzoïque
- 5-[3-hydroxy-3-(5,6,7,8-tétrahydro-5,5,8,8-tétraméthyl-2-naphtyl)-1-propynyl]2-thiophènecarboxylate de méthyle
- 2-hydroxy-4-[3-hydroxy-3-(5,6,7,8-tétrahydro-3,5,5,8,8-pentaméthyl-2-naphtyl)-1-propynyl]benzoate de méthyle
- Acide 2-hydroxy-4-[3-hydroxy-3-(5,6,7,8-tétrahydro-3,5,5,8,8-pentaméthyl-2-naphtyl)-1-propynyl]benzoïque
- 2-hydroxy-4-[3-hydroxy-3-(3-méthoxy-5,6,7,8-tétrahydro-5,5,8,8-tétraméthyl-2-naphtyl)-1-propynyl]benzoate de méthyle
- Acide 2-hydroxy-4-[3-hydroxy-3-(3-méthoxy-5,6,7,8-tétrahydro-5,5,8,8-tétraméthyl-2-naphtyl)-1-propynyl]benzoïque
- Acide 4-[3-hydroxy-3-(4,4-diméthylthiochroman-6-yl)-1-propynyl]benzoïque
- Acide 4-[3-hydroxy-3-(4,4-diméthylthiochroman-7-yl)-1-propynyl]benzoïque
- 3-méthyl-4-[3-hydroxy-3-(5,6,7,8-tétrahydro-5,5,8,8-tétraméthyl-2-naphtyl)-1-propynyl]benzoate de méthyle
- Acide 3-méthyl-4-[3-hydroxy-3-(5,6,7,8-tétrahydro-5,5,8,8-tétraméthyl-2-naphtyl)-1-propynyl]benzoïque
- Acide 2-chloro-4-[3-hydroxy-3-(5,6,7,8-tétrahydro-5,5,8,8-tétraméthyl-2-naphtyl)-1-propynyl]benzoïque
- Acide 2-acétoxy-4-[3-acétoxy-3-(5,6,7,8-tétrahydro-5,5,8,8-tétraméthyl-2-naphtyl)-1-propynyl]benzoïque
- 4-[3-hydroxy-3-(3-tert-butyl-4-propyloxyphényl)-1-propynyl]benzoate de méthyle
- 4-[3-hydroxy-3-(3-tert-butyl-4-héxyloxyphényl)-1-propynyl]benzoate de méthyle

Selon la présente invention, les composés de formule (I) plus particulièrement préférés sont ceux pour lesquels $R_5$ représente -OH, $R_7$ représente un radical $OR_8$, et $R_{11}$ représente un radical $-OR_6$, $R_6$ et $R_{11}$ ayant la signification donnée ci-avant.

La présente invention a également pour objet les procédés de préparation des composés de formule (I) ci-dessus selon les schémas réactionnels donnés aux Figures 1, 2 et 3.

Les dérivés de formule (Ia) peuvent être préparés par une suite de réactions comprenant l'action d'un chlorure de benzoyle de formule (1) avec un dérivé acétylénique de formule (2) en présence d'un acide de Lewis (par exemple $AlCl_3$) dans un solvant chloré, tel le dichlorométhane.La cétone (3) ainsi obtenue est réduite en alcool (4) par action d'un hydrure alcalin, tel le borohydrure de sodium, dans un solvant alcoolique (par exemple le méthanol). Puis saponification de la fonction ester en présence d'une base, telle l'hydroxyde de sodium ou de lithium dans un solvant alcoolique ou dans le THF.

Les dérivés de formule (Ib) sont obtenus par oxydation du dérivé (Ia) en présence de pyridinium dichromate ou d'oxyde de manganèse dans un solvant organique tel le dichlorométhane.

Les dérivés de formule (Ic) peuvent être obtenus par couplage d'un dérivé halogéné (4), de préférence iodé ou bromé, avec un dérivé α-hydroxy acétylénique (3) en présence d'un catalyseur au Palladium [par exemple le chlorure de Bis-(triphénylphosphine)-palladium(II)] dans un solvant, tel la triéthylamine. Le dérivé α-hydroxy acétylénique (3) est obtenu par action d'un chlorure de benzoyle de formule (1) avec le triméthylsilylacétylène en présence d'un acide de Lewis (par exemple $AlCl_3$) dans un solvant chloré, puis réduction de la cétone obtenu (2) avec un hydrure alcalin (par exemple le borohydrure de sodium) dans un solvant alcoolique.

Les dérivés de formule (Ic) peuvent être aussi obtenus lorsque $R_1$ est différent de $-COOR_8$ par action d'un phényl acétylénure de lithium de formule (6) avec un dérivé benzaldéhyde de formule (5) dans un solvant organique, tel l'éther éthylique ou le THF.

Les dérivés de formule (Id) peuvent être préparés par action d'un acétylénure de bore (3) (préparé in situ à partir de phényl acétylénure de lithuim et de trifluorure de bore à -78°C dans le THF) avec un benzamide tertiaire de formule (4) dans un solvant organique, tel le THF. Par réduction du composé précédent avec un hydrure alcalin on obtient les composés de formule (Ie).

Lorsque $R_1$ représente un radical -COOH et $R_{10}$, $R_{11}$ pris ensemble forment un radical oxo, les composés sont préférentiellement préparés en protégeant $R_1$ sous forme d'ester méthylique, éthylique ou allylique, le passage à la forme libre étant éffectué en présence d'hydroxyde de lithium dans le THF.

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités des composés respectivement dans les domaines de la différenciation et de la prolifération cellulaire.

Les composés selon l'invention conviennent particulièrement bien dans les domaines de traitement suivants :

1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine $D_2$ ou $D_3$ et en particulier la 1,25-dihydroxyvitamine $D_3$. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0.001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0.001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzylcystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoides et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner à titre d'illustration et sans aucun caractère limitatif, plusieurs exemples d'obtention de composés actifs de formule (I) selon l'invention, ainsi que diverses formulations concrètes à base de tels composés.

## EXEMPLE 1

*4-[3-oxo-3-(5,6,7,8-tétrahydro-5,5,8,8-tétraméthyl-2-naphtyl)-1-propynyl]benzoate de méthyle.*

(a) 4-triméthylsilyléthynylbenzoate de méthyle.

Dans un tricol et sous courant d'azote , on introduit 21,5 g (0,1 mole) de 4-bromobenzoate de méthyle, 300 ml de triéthylamine et un mélange de 200 mg d'acétate de palladium et de 400 mg de triphénylphosphine. On ajoute ensuite 20 g (0,204 mole)de triméthylsilylacétylène, chauffe progressivement à 90°C durant 1 heure et laisse à cette température pendant 5 heures. On refroidit le milieu réactionnel, filtre le sel et évapore. On reprend le résidu avec 200 ml d'acide chlorhydrique (5%) et 400 ml d'éther éthylique. On décante la phase éthèrée, lave à l'eau, sèche sur sulfate de magnésium, évapore. Le résidu obtenu est purifié par chromatographie sur colonne de silice, élué avec du dichlorométhane. Après évaporation des solvants, on recueille 23 g (100%) du dérivé attendu sous forme d'une huile incolore.

(b) 4-[3-oxo-3-(5,6,7,8-tétrahydro-5,5,8,8-tétraméthyl-2-naphtyl)-1-propynyl]benzoate de méthyle.

Dans un ballon, on introduit 8,4 g (36 mmoles) de chlorure de 5,6,7,8-tétrahydro-5,5,8,8-tetraméthyl-2-naphtoyle 6,9 g (29,7 mmoles) du dérivé précédent et 100 ml de dichlorométhane. On ajoute à 0°C par petites quantités 16 8 g (125 mmoles) d'$AlCl_3$ et agite à température ambiante pendant 8 heures. On verse le milieu réactionnel dans la glace, extrait avec du dichlorométhane, décante la phase organique, sèche sur sulfate de magnésium, évapore. Le résidu obtenu est purifié par chromatographie sur colonne de silice élué avec un mélange de dichlorométhane et d'hexane (50-50). On recueille 6,8 g (61%) de produit attendu, de point de fusion 113-4°C.

## EXEMPLE 2

*4-[3-hydroxy-3-(5,6,7,8-tétrahydro-5,5,8,8-tétraméthyl-2-naphtyl)-1-propynyl]benzoate de méthyle.*

Dans un ballon, on introduit 4,7 g (125 mmoles) du produit obtenu à l'exemple 1(b) et 100 ml de méthanol. Tout en refroidissant à 0°C, on ajoute successivement 5,7 g (150 mmoles) de $CeCl_3$, $7H_2O$ et 530 mg (125 mmoles) de borohydrure de sodium et agite à température ambiante pendant 4 heures. On verse le milieu réactionnel dans un mélage eau-éther éthylique, décante la phase organique, lave à l'eau, sèche sur sulfate de magnésium, évapore. Le résidu obtenu est trituré dans 100 ml d'hexane, filtré et sèché. On recueille 4 g (85%) du produit attendu de point de fusion 142-3°C

## EXEMPLE 3

*Acide 4-[3-hydroxy-3-(5,6,7,8-tétrahydro-5,5,8,8-tétraméthyl-2-naphtyl)-1-propynyl]benzoïque.*

Dans un ballon, on introduit 3,5 g (93 mmoles) de 4-[3-hydroxy-3-(5,6,7,8-tétrahydro-5,5,8,8-tétraméthyl-2-naphtyl)-1-propynyl]benzoate de méthyle, 200 ml de méthanol et 20 ml d'une solution de soude méthanolique (2N). On agite à température ambiante pendant 8 heures, évapore le milieu réactionnel, reprend le résidu par l'eau, acidifie avec de l'acide chlorhydrique. On extrait avec de l'éther éthylique, décante la phase organique, sèche sur sulfate de magnésium et évapore. Le résidu obtenu est recristallisé dans un mélange cyclohexane-éther isopropylique, on recueille 1,7 g (50%) de l'acide attendu de point de fusion 134-5°C.

**EXEMPLE 4**

*Acide 4-[3-oxo-3-(5,6,7,8-tétrahydro-5,5,8,8-tétraméthyl-2-naphtyl)-1-propynyl]benzoïque.*

On introduit 500 mg (1,38 mmoles) d'acide 4-[3-hydroxy-3-(5,6,7,8-tétrahydro-5,5,8,8-tétraméthyl-2-naphtyl)-1-propynyl]benzoïque, 50 ml de dichlorométhane et 2,4 g (27,6 mmoles) d'oxyde de manganèse dans un ballon que l'on place dans un bain à ultra-son pendant 4 heures. On filtre le milieu réactionnel, évapore le filtrat et purifie le résidu par simple filtration sur silice dans l'éther éthylique. On recueille 90 mg (18%) du produit attendu de point de fusion 208-209°C.

**EXEMPLE 5**

*Acide 2-hydroxy-4-[3-oxo-3-(5,6,7,8-tétrahydro-5,5,8,8-tétraméthyl-2-naphtyl)-1-propynyl]benzoïque.*

(a) 2-hydroxy-4-triméthylsilyléthynylbenzoate de méthyle

De manière analogue à l'exemple 1(a) par réaction de 34 g (122 mmoles) de 2-hydroxy-4-iodobenzoate de méthyle avec 34 ml (244 mmoles) de triméthylsilylacétylène. on obtient 25,9 g (85%) de produit attendu sous forme d'une huile marron.

(b) 2-hydroxy-4-[3-oxo-3-(5,6,7,8-tétrahydro-5,5,8,8-tétraméthyl-2-naphtyl)-1-propynyl]benzoate de méthyle.

De manière analogue à l'exemple 1(b), par réaction de 2,4g ((0,01 mole)de chlorure de 5,6,7,8-tétrahydro-5,5,8,8-tétraméthyl-2-naphtoyle avec 2,5 g (0,01 mole) de 2-hydroxy-4-triméthylsilyléthynylbenzoate de méthyle, on obtient 2,9 g (74%) de l'ester attendu de point de fusion 189-190°C.

(c) Acide 2-hydroxy-4-[3-oxo-3-(5,6,7,8-tétrahydro-5,5,8,8-tétraméthyl-2-naphtyl)-1-propynyl]benzoïque.

Dans un ballon, on introduit 1,5 g (3,8 mmoles) de l'ester précédent. 100 ml de THF et 485 mg (11,4 mmoles) d'hydroxyde de lithium monohydrate. On chauffe à reflux pendant 8 heures, évapore à sec, reprend par l'eau, acidifie avec de l'acide chlorhydrique. On extrait avec de l'éther éthylique, décante la phase organique, sèche sur sulfate de magnésium, évapore. On recristallise le résidu obtenu dans un mélange de cyclohexane et d'éther isopropylique, filtre et sèche. On recueille 700 mg (48%) d'acide attendu de point de fusion 183-4°C.

**EXEMPLE 6**

*2-hydroxy-4-[3-hydroxy-3-(5,6,7,8-tétrahydro-5,5,8,8-tétraméthyl-2-naphtyl)-1-propynyl]benzoate de méthyle.*

Dans un ballon, on introduit 2,9 g (7,4 mmoles) de 2-hydroxy-4-[3-oxo-3-(5,6,7,8-tétrahydro-5,5,8,8-tétraméthyl-2-naphtyl)-1-propynyl)benzoate de méthyle, 100 ml d'un mélange méthanol-THF (50-50) et ajoute par petites quantités 140 mg (3,7 mmoles) de borohydrure de sodium. On agite à température ambiante pendant 2 heures, verse le milieu réactionnel dans l'eau, extrait avec de l'éther éthylique, décante la phase organique, sèche sur sulfate de magnésium et évapore.Le résidu obtenu est purifié par chromatographie sur colonne de silice élué avec un mélange de dichlorométhane et d'hexane (50-50) On recueille après évaporation des solvants 1,6 g (55%) de produit attendu de point de fusion 92-3°C..

**EXEMPLE 7**

*Acide 2-hydroxy-4-[3-hydroxy-3-(5,6,7,8-tétrahydro-5,5,8,8-tétraméthyl-2-naphtyl)-1-propynyl]benzoïque.*

De manière analogue à l'exemple 6 à partir de 1 g (2,7 mmoles) de l'acide 2-hydroxy-4-[3-oxo-3-(5,6,7,8-tétrahydro-5,5,8,8-tétraméthyl-2-naphtyl)-1-propynyl]benzoïque, on obtient 915 mg (91 %) de l'acide attendu de point de fusion 203-4°C.

**EXEMPLE 8**

*Acide 2-hydroxy-4-[3-oxo-3-(3-tert-butyl-4-méthoxyphényl)-1-propynyl]benzoïque.*

(a) 2-hydroxy-4-[3-oxo-3-(3-tert-butyl-4-méthoxyphényl)-1-propynyl]benzoate de méthyle.

De manière analogue à l'exemple 1(b) par réaction de 4,2 g (0,02 mole) de chlorure de 3-tert-butyl-4-méthoxybenzoyle avec 5 g (0,02 mole) de 2-hydroxy-4-triméthylsilyléthynylbenzoate de méthyle, on obtient après purification par chromatographie sur colonne de silice élué avec du dichlorométhane 6 g (81 %) de produit attendu sous forme d'une huile marron.

(b) Acide 2-hydroxy-4-[3-oxo-3-(3-tert-butyl-4-méthoxyphényl)-1-propynyl]benzoïque.

De manière analogue à l'exemple 5(c) à partir de 6 g (16,4 mmoles) du produit précédent, on obtient 4,2 g (73%) de l'acide attendu de point de fusion 204-5°C.

**EXEMPLE 9**

*Acide 4-[1-oxo-3-(5,6,7,8-tétrahydro-5,5,8,8-tétraméthyl-2-naphtyl)-2-propynyl]benzoïque*

a) 5,6,7,8-tétrahydro-5,5,8,8-tétraméthyl-2-triméthylsilylethynylnaphtalène

De manière analogue à l'exemple 1(a) par réaction de 26,7 g (0,1 mole) de 2-bromo-5,6,7,8-tétrahydro-5,5,8,8-tétraméthylnaphtalène avec 20 g (0,204 mole) de triméthylsilylacétylène, on obtient 18,8 g(66%) de produit attendu sous forme d'une huile incolore.

(b) 5,6,7,8-tétrahydro-5,5,8,8-tétraméthyl-6-ethynylnaphtalène

Dans un ballon, on introduit 5,7 g (0,02 mole) du produit précédent, 75 ml de méthanol et ajoute 100 mg de carbonate de potassium. On agite à température ambiante pendant 3 heures, évapore à sec, reprend le résidu par l'eau et l'éther éthylique, décante la phase organique, sèche sur sulfate de magnésium et évapore.On recueille 4,1 g (100%) d'acétylènique attendu sous forme d'une huile jaune.

(c) 4-N,N'diméthylcarbamoylbenzoate d'allyle

Dans un ballon, on introduit 20 ml de diméthylamine (40% dans l'eau) et ajoute goutte à goutte une solution de 2,5 g (11,6 mmoles) de chlorure de 4-(allyloxycarbonyl)benzoyle dans 50 ml de THF et agite à température ambiante pendant 1 heure. On verse le milieu réactionnel dans l'eau, extrait avec de l'éther éthylique, décante la phase organique, sèche sur sulfate de magnésium, évapore. On recueille 2,7 g (100%) de l'amide attendu sous forme d'une huile légèrement jaune.

(d) 4-[1-oxo-3-(5,6,7,8-tétrahydro-5,5,8,8-tétraméthyl-2-naphtyl)-2-propynyl]benzoate d'allyle

Dans un tricol, on introduit,sous courant d'azote, 4,3 g (20 mmoles) de 5,6,7,8-tétrahydro-5,5,8,8-tétraméthyl-6-ethynylnaphtalène et 20 ml de THF. A -78°C, on ajoute goutte à goutte 12,5 ml (20 mmoles) d'une solution de n-butyllithium (1,6M dans l'hexane) et agite pendant 10 minutes. A cette même température, on ajoute ensuite 2,7 ml de $BF_3$-$Et_2O$ et agite pendant 30 minutes. A cette solution, toujours à -78°C, on ajoute une solution de 2,5 g (10 mmoles) de 4-N,N'diméthylcarbamoylbenzoate d'allyle dans 10 ml de THF et agite pendant 1 heure. On verse le milieu réactionnel dans une solution aqueuse de chlorure d'ammonium, extrait avec de l'éther éthylique, décante la phase organique, sèche sur sulfate de magnésium et évapore. Le résidu obtenu est purifié par chromatographie sur colonne de silice élué avec un mélange de dichlorométhane et d'hexane (10-90). Après évaporation des solvants, on recueille 4,2 g (52%) de l'ester attendu sous forme d'une huile.

(e) Acide 4-[1-oxo-3-(5,6,7,8-tétrahydro-5,5,8,8-tétraméthyl-2-naphtyl)-2-propynyl]benzoïque

De manière analogue à l'exemple 5(c), à partir de 1,4 g (3,5 mmoles) de l'ester précédent, on obtient 940 mg (75%) de l'acide attendu de point de fusion 191-2°C.

**EXEMPLE 10**

*Acide 4-[1-hydroxy-3-(5,6,7,8-tétrahydro-5,5,8,8-tétraméthyl-2-naphtyl)-2-propynyl]benzoïque*

(a) 4-[1-hydroxy-3-(5,6,7,8-tétrahydro-5,5,8,8-tétraméthyl-2-naphtyl)-2-propynyl]benzoate d'allyle

De manière analogue à l'exemple 6, à partir de 1,7 g (4,2 mmoles) de 4-[1-oxo-3-(5,6,7,8-tétrahydro-5,5,8,8-tétraméthyl-2-naphtyl)-2-propynyl]benzoate d'allyle, on obtient 1,6 g (94%) de l'ester attendu sous forme d'une huile incolore.

(b) Acide 4-[1-hydroxy-3-(5,6,7,8-tétrahydro-5,5,8,8-tétraméthyl-2-naphtyl)-2-propynyl]benzoïque

Dans un ballon et sous courant d'azote, on introduit 950 mg (2,4 mmoles) de l'ester précédent, 50 ml de THF et 75 mg de tétrakis(triphénylphosphine)palladium(0). A 0°C, on ajoute goutte à goutte 2,1 ml (24 mmoles)de morpholine et agite à température ambiante pendant 1 heure. On évapore le milieu réactionnel, reprend le résidu par l'eau, acidifie à pH 1 avec de l'acide chlorhydrique, extrait avec de l'éther éthylique, décante la phase organique, sèche sur sulfate de magnésium et évapore.Le résidu obtenu est trituré dans un mélange hexane-éther éthylique (80-20), filtré et séché. On recueille 530 mg (62%) d'acide attendu de point de fusion 161-2°C.

**EXEMPLE 11**

*2-hydroxy-4-[3-hydroxy-3-(4,4-diméthylthiochroman-6-yl)-1-propynyl]benzoate de méthyle.*

(a) 4,4-diméthyl-6-thiochromancarboxaldéhyde.

Dans un tricol et sous courant d'azote, on introduit 9,4 g (36,6 mmoles) de 4,4-diméthyl-6-bromothiochroman et 100 ml de THF. A -78°C on ajoute goutte à goutte 16,1 ml d'une solution de n-butyllithium (2,5 M dans l'hexane) et agite 30'. On ajoute ensuite goutte à goutte 2,7 ml (38,4 mmoles) de DMF et laisse remonter à température ambiante. On verse le milieu réactionnel dans une solution aqueuse de chlorure d'ammonium, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (50-50). Après évaporation des solvants, on recueille 6,1 g (81 %) d'aldéhyde attendu sous forme d'une huile jaune.

(b) α-Triméthylsilyléthynyl-(4,4-diméthyl-6-thiochroman)méthanol.

Dans un tricol, on introduit 3 ml (21,3 mmoles) de triméthylsilylacétylène et 50 ml de THF. A -78°C et sous courant d'azote, on ajoute goutte à goutte une solution de 8,6 ml (21,3 mmoles) de n-butyllithium (2,5 M dans l'hexane) et laisse revenir à température ambiante.
Cette solution est introduite goutte à goutte dans une solution de 4 g (19,4 mmoles)de 4,4-diméthyl-6-thiochromancarboxaldéhyde dans 50 ml de THF à -78°C. On laisse le milieu réactionnel revenir à température ambiante, le verse dans une solution aqueuse de chlorure d'ammonium, extrait avec de l'éther éthylique, décante la phase organique, sèche sur sulfate de magnésium, évapore. On obtient 5,9 g (100%) de l'alcool attendu sous forme d'une huile jaune.

(c) α-éthynyl-(4,4-diméthyl-6-thiochroman)méthanol.

Dans un ballon, on introduit 5,9 g (19,4 mmoles) d'a-Triméthylsilyléthynyl-(4,4-diméthyl-6-thiochroman)méthanol 50 ml de THF et ajoute goutte à goutte 21,3 ml (23,3 mmoles) d'une solution de fluorure de tétrabutylammonium (1,1 M dans le THF). On agite à température ambiante une heure, verse le milieu réactionnel dans l'eau, extrait avec de l'éther éthylique, décante la phase organique, sèche sur sulfate de magnésium, évapore.Le résidu obtenu est purifié par chromatographie sur colonne de silice élué avec un mélange d'acétate d'éthyle et d'hexane (1-4). Après évaporation des solvants, on recueille 3,9 g (87%) d'α-éthynyl-(4,4-diméthyl-6-thiochroman)méthanol sous forme d'une huile incolore.

(d) 2-hydroxy-4-[3-hydroxy-3-(4,4-diméthylthiochroman-6-yl)-1-propynyl]benzoate de méthyle.

Dans un tricol, on introduit 2,5 g (10,8 mmoles) d'α-éthynyl-(4,4-diméthyl-6-thiochroman)méthanol, 3 g (10,8 mmoles) de 2-hydroxy-4-iodobenzoate de méthyle et 50 ml de triéthylamine. On dégaze le milieu réactionnel avec de l'azote pendant 30', puis ajoute successivement 600 mg (0,86 mmoles) de Bis(triphénylphosphine)palladium(II)chlorure et 240

mg (1,3 mmole) d'iodure de cuivre. On agite à température ambiante pendant quatre heures, évapore à sec le milieu réactionnel, reprend le résidu obtenu par l'eau et l'éther éthylique. On décante la phase organique, sèche sur sulfate de magnésium, évapore. Le résidu obtenu est purifié par chromatographie sur colonne de silice élué avec du dichlorométhane, on recueille 3,2 g (80%) de 2-hydroxy-4-[3-hydroxy-3-(4,4-diméthylthiochroman-6-yl)-1-propynyl]benzoate de méthyle de point de fusion 105-6°C.

**EXEMPLE 12**

*Acide 2-hydroxy-4-[3-oxo-3-(4,4-diméthylthiochroman-6-yl)-1-propynyl/benzoïque.*

(a) 2-hydroxy-4-[3-oxo-3-(4,4-diméthylthiochroman-6-yl)-1-propynyl]benzoate de méthyle.

Dans un ballon, on introduit 2 g (5,2 mmoles) de 2-hydroxy-4-[3-hydroxy-3-(4,4-diméthylthiochroman-6-yl)-1-propynyl]benzoate de méthyle et 50 ml de dichlorométhane. On ajoute 2,6 g (6,9 mmoles) de pyridinium dichromate et agite à température ambiante 8 heures. On évapore le milieu réactionnel à sec et purifie le résidu obtenu par chromatographie sur colonne de silice élué avec un mélange de dichlorométhane et d'hexane (50-50). Après évaporation des solvants on recueille 1,3 g (65%) de l'ester attendu sous forme d'une huile brune.

(b) acide 2-hydroxy-4-[3-oxo-3-(4,4-diméthylthiochromam-6-yl)-1-propynyl]benzoïque.

Dans un ballon, on introduit 1,3 g (3,42 mmoles) de l'ester précédent, 430 mg (10,2 mmoles) d'hydroxyde de lithium et 50 ml de THF. On chauffe à reflux pendant 8 heures, évapore à sec le milieu réactionnel. Le résidu est repris dans l'eau et l'éther éthylique et acidifié. On décante la phase organique, sèche sur sulfate de magnésium, évapore.Le résidu obtenu est purifié par chromatographie sur colonne de silice élué avec un mélange de dichlorométhane et de méthanol (95-5). On recueille 600 mg (48%) de l'acide attendu de point de fusion 253-4°C.

**EXEMPLE 13**

*Acide 4-[3-hydroxy-3-(3-tert-butyl-4-méthoxyphényl-1-propynyl]benzoïque.*

(a) α-Triméthylsilyléthynyl-(3-tert-butyl-4-méthoxybenzène)méthanol.

De manière analogue à l'exemple 11(b) par réaction de 7,7 g (40 mmoles) de 3-tert-butyl-4-méthoxybenzaldéhyde avec un équivalent de triméthylsilylacétylénure de lithium, on obtient 11,1 g (98%) d'alcool attendu sous forme d'une huile incolore.

(b) α-éthynyl-(3-tert-butyl-4-méthoxybenzène)éthanol.

De manière analogue à l'exemple 11(c) à partir de 11,1 g (38,2 mmoles) d'α-Triméthylsilyléthynyl-(3-tert-butyl-4-méthoxybenzène)méthanol, on obtient 8,1 g (96%) d'α-éthynyl-(3-tert-butyl-4-méthoxybenzène)méthanol sous forme d'une huile jaune.

(c) 4-[3-hydroxy-3-(3-tert-butyl-4-méthoxyphényl)-1-propynyl]benzoate de méthyle.

De manière analogue à l'exemple 11(d) par réaction de 2,5 g (11,4 mmoles) d'α-éthynyl-(3-tert-butyl-4-méthoxybenzène)méthanol avec 3,17 g (11,4 mmoles) de 2-hydroxy-4-iodobenzoate de méthyle, on obtient 3 g (71%) de l'ester attendu sous forme d'une huile brune.

(d) acide 4-[3-hydroxy-3-(3-tert-butyl-4-méthoxyphényl)-1-propynyl]benzoïque.

De manière analogue à l'exemple 12(b) à partir de 4,5 g (12,8 mmoles) de 4-[3-hydroxy-3-(3-tert-butyl-4-méthoxyphényl)-1-propynyl]benzoate de méthyle, on obtient 2,45 g (57%) de l'acide attendu de point de fusion 114-5°C.

**EXEMPLE 14**

*N-méthyl-4-[3-hydroxy-3-(5,6,7,8-tétrahydro-5,5,8,8-tétraméthyl-2-naphtyl)-1-propynyl]-2-pyrrole carboxylate de méthyle.*

(a) α-Triméthylsilyléthynyl-(5,6,7,8-tétrahydro-5,5,8,8-tétraméthyl-2-naphtalène) méthanol.

Dans un tricol, on introduit 17,13 ml (0,121 mole) de triméthylsilylacétylène et 100 ml de THF. A -78°C et sous courant d'azote, on ajoute goutte à goutte une solution de 48,5 ml (0,121 mole) de n-butyllithium (2,5 M dans l'hexane) et laisse revenir à température ambiante.
Cette solution est introduite goutte à goutte dans une solution de 23,8 g (0,11 mole)de 5,6,7,8-tétrahydro-5,5,8,8-tétraméthyl-2-naphtalènecarboxaldéhyde dans 100 ml de THF à -78°C. On laisse le milieu réactionnel revenir à température ambiante, le verse dans une solution aqueuse de chlorure d'ammonium, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (50-50). Après évaporation des solvants, on recueille 29,9 g (86%) de l'alcool attendu sous forme d'une huile jaune.

(b) α-éthynyl-(5,6,7,8-tétrahydro-5,5,8,8-tétraméthyl-2-naphtalène)méthanol.

Dans un ballon, on introduit 29,9 g (95,2 mmoles) d'α-Triméthylsilyléthynyl-(5,6,7,8-tétrahydro-5,5,8,8-tétraméthyl-2-naphtalène)méthanol 100 ml de THF et ajoute goutte à goutte 103,8 ml (114,2 mmoles) d'une solution de fluorure de tétrabutylammonium (1,1 M dans le THF). On agite à température ambiante une heure, verse le milieu réactionnel dans l'eau, extrait avec de l'éther éthylique, décante la phase organique, sèche sur sulfate de magnésium, évapore.Le résidu obtenu est purifié par chromatographie sur colonne de silice élué avec un mélange d'acétate d'éthyle et d'hexane (1-4). Après évaporation des solvants, on recueille 18,1 g (79%) d'α-éthynyl-(5,6,7,8-tétrahydro-5,5,8,8-tétraméthyl-2-naphtalène)méthanol de point de fusion 56-7°C.

(c) 2-trichloroacétylpyrrole.

Dans un tricol, on introduit 45 g (247 mmoles) de chlorure de trichloroacétyle et 100 ml d'éther éthylique. On ajoute goutte à goutte une solution de 15,4 g (230 mmoles) de pyrrole dans 100ml d'éther éthylique et agite à température ambiante une heure, on ajoute ensuite lentement une solution de 20 g de carbonate de potassium dans 60 ml d'eau. On décante la phase organique, sèche sur sulfate de magnésium, évapore, triture le résidu dans l'hexane et filtre. On recueille 42,7 g (87%) de produit attendu de point de fusion 78-9°C.

(d) 4-iodo-2-trichloroacétylpyrrole.

Dans un tricol et sous courant d'azote, on introduit 8,4 g (39,5 mmoles) de 2-trichloroacétylpyrrole et 100 ml de chloroforme et ajoute sucessivement 8,8 g (39,5 mmoles) de trifluoroacétate d'argent et 10,16 g (39,5 mmoles) d'iode. On agite à température ambiante pendant une heure, verse le milieu réactionnel dans la glace, extrait avec du dichlorométhane, décante la phase organique, sèche sur sulfate de magnésium, évapore. Le résidu obtenu est trituré dans l'hexane et filtré, on recueille 8,2 g (61%) du produit attendu de point de fusion 118-9°C.

(e) 4-iodo-2-pyrrolecarboxylate de méthyle.

Dans un ballon, on introduit 8,2 g (24 mmoles) de 4-iodo-2-trichloroacétyl pyrrole 100 ml de méthanol et ajoute 2 g (36 mmoles) de méthylate de sodium. On agite à température ambiante pendant quatre heures, évapore à sec le milieu réactionnel,reprend le résidu obtenu par l'eau et l'éther éthylique, décante la phase organique, sèche sur sulfate de magnésium, évapore. Le résidu obtenu est trituré dans l'heptane, filtré, on recueille 4,9 g (81%) de l'ester attendu de point de fusion 77-8°C.

(f) N-méthyl-4-iodo-2-pyrrolecarboxylate de méthyle.

Dans un tricol on introduit 780 mg (25,9 mmoles) d'hydrure de sodium (80% dans l'huile) et 20 ml de DMF, on ajoute goutte à goutte une solution de 6,5 g (25,9 mmoles) de 4-iodo-2-pyrrolecarboxylate de méthyle dans 50 ml de DMF et agite jusqu'à cessation du dégagement gazeux. On ajoute ensuite 2,1 ml (33,6 mmoles) d'iodométhane et agite à température ambiante deux heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (40-60). On recueille 4,5 g (65%) de N-méthyl-4-iodo-2-pyrrolecarboxylate de méthyle de point de fusion 64-5°C.

(g) N-méthyl-4-[3-hydroxy-3-(5,6,7,8-tétrahydro-5,5,8,8-tétraméthyl-2-naphtyl)-1-propynyl]-2-pyrrole carboxylate de méthyle.

De manière analogue à l'exemple 11(d) par réaction de 2,9 g (12 mmoles) de α-éthynyl-(5,6,7,8-tétrahydro-5,5,8,8-tétraméthyl-2-naphtaléne)méthanol avec 3,2 g (12,1 mmoles) de N-méthyl-4-iodo-2-pyrrolecarboxylate de méthyle, on recueille par trituration dans l'éther isopropylique 2,8 g (61%) de l'ester attendu de point de fusion 150-2°C.

**EXEMPLE 15**

*Acide N-méthyl-4-[3-hydroxy-3-(5,6,7,8-tétrahydro-5,5,8,8-tétraméthyl-2-naphtyl)-1-propynyl]-2-pyrrole carboxylique.*

De manière analogue à l'exemple 12(b) à partir de 2 g (5,2 mmoles) de N-méthyl-4-[3-hydroxy-3-(5,6,7,8-tétrahydro-5,5,8,8-tétraméthyl-2-naphtyl)-1-propynyl]-2-pyrrole carboxylate de méthyle, on obtient 440 mg (22%) de l'acide attendu de point de fusion 112-3°C.

**EXEMPLE 16**

*4-[3-hydroxy-3-(5,6,7,8-tétrahydro-5,5,8,8-tétraméthyl-2-naphtyl)-1-propynyl]-2-pyrrole carboxylate de méthyle.*

(a) N-tert-butoxycarbonyl-4-iodo-2-pyrrolecarboxylate de méthyle.

Dans un tricol on introduit 780 mg (25,9 mmoles) d'hydrure de sodium (80% dans l'huile) et 20 ml de DMF, on ajoute goutte à goutte une solution de 6,5 g (25,9 mmoles) de 4-iodo-2-pyrrolecarboxylate de méthyle dans 50 ml de DMF et agite jusqu'à cessation du dégagement gazeux. On ajoute ensuite par petites quantités 5,6 g (25.9 mmoles) de di-tert-butyldicarbonate et agite à température ambiante deux heures. On verse le milieu réactionnel dans l'eau, extrait avec de l'éther éthylique, décante la phase organique,sèche sur sulfate de magnésium, évapore. Le résidu obtenu est purifié par chromatographie sur colonne de silice élué avec un mélange de dichlorométhane et d'hexane (60-40). On recueille 6,8 g (75%) de N-tert-butoxycarbonyl-4-iodo-2-pyrrolecarboxylate de méthyle sous forme d'une huile jaune.

(b) N-tert-butoxycarbonyl-4-[3-hydroxy-3-(5,6,7,8-tétrahydro-5,5,8,8-tétraméthyl-2-naphtyl)-1-propynyl]-2-pyrrole carboxylate de méthyle.

De manière analogue à l'exemple 11(d) par réaction de 2 g (8,2 mmoles) de α-éthynyl-(5,6,7,8-tétrahydro-5,5,8,8-tétraméthyl-2-naphtalène)méthanol avec 3 g (8,5 mmoles) de N-tert-butoxycarbonyl-4-iodo-2-pyrrolecarboxylate de méthyle, on obtient 3,8 g (98%) de l'ester attendu sous forme d'une huile brune.

c) 4-[3-hydroxy-3-(5,6,7,8-tétrahydro-5,5,8,8-tétraméthyl-2-naphtyl)-1-propynyl]-2-pyrrole carboxylate de méthyle.

Dans un ballon on introduit 2,4 g (5,1 mmoles) de l'ester précédent 20 ml de THF et 20 ml de méthanol. On ajoute 278 mg (5,1 mmoles) de méthylate de sodium et agite à température ambiante pendant quatre heures. On évapore à sec le milieu réactionnel, reprend par l'eau et l'éther éthylique, décante la phase organique, sèche sur sulfate de magnésium, évapore. Le résidu obtenu est trituré dans un mélange d'éther diisopropylique et d'heptane, filtré. On recueille 1,22 g (65%) de l'ester attendu de point de fusion 95-100°C.

**EXEMPLE 17**

*2-hydroxy-4-[3-hydroxy-3-(4,4-diméthylthiochroman-7-yl)-1-propynyl]benzoate de méthyle.*

(a) 4,4-diméthyl-7-thiochromancarboxaldéhyde.

De manière analogue à l'exemple 11(a) à partir de 5,6 g (21,8 mmoles) de 4,4-diméthyl-7-bromothiochroman, on obtient 3,3 g (74%) d'aldéhyde attendu sous forme d'une huile jaune.

(b) α-Triméthylsilyléthynyl-(4,4-diméthyl-7-thiochroman)méthanol.

De manière analogue à l'exemple 11(b) à partir de 5,4 g (26,2 mmoles)de 4,4-diméthyl-7-thiochromancarboxaldéhyde, on obtient 8 g (100%) d'α-triméthylsilyl éthynyl-(4,4-diméthyl-7-thiochroman)méthanol sous forme d'une huile jaune.

(c) α-éthynyl-(4,4-diméthyl-7-thiochroman)méthanol.

De manière analogue à l'exemple 11(c) à partir de 8 g (26,3 mmoles) d'α-Triméthylsilyléthynyl-(4,4-diméthyl-7-thiochroman)méthanol, on obtient après purification 4,3 g (70%) d'alcool attendu sous forme d'une huile incolore.

(d) 2-hydroxy-4-[3-hydroxy-3-(4,4-diméthylthiochroman-7-yl)-1-propynyl]benzoate de méthyle.

Dans un tricol, on introduit 2,5 g (10,8 mmoles) d'α-éthynyl-(4,4-diméthyl-7-thiochroman)méthanol, 3 g (10,8 mmoles) de 2-hydroxy-4-iodobenzoate de méthyle et 50 ml de triéthylamine. On dégaze le milieu réactionnel avec de l'azote pendant 30', puis ajoute sucessivement 600 mg (0,86 mmoles) de Bis(triphénylphosphine)palladium(II)chlorure et 240 mg (1,3 mmoles) d'iodure de cuivre. On agite à température ambiante pendant quatre heures, évapore à sec le milieu réactionnel, reprend le résidu obtenu par l'eau et l'éther éthylique. On décante la phase organique, sèche sur sulfate de magnésium, évapore. Le résidu obtenu est purifié par chromatographie sur colonne de silice élué avec du dichlorométhane, on recueille 3,2 g (80%) de 2-hydroxy-4-[3-hydroxy-3-(4,4-diméthylthiochroman-7-yl)-1-propynyl]benzoate de méthyle de point de fusion 101-2°C.

**EXEMPLE 18**

*Acide 4-[1-hydroxy-3-(4,4-diméthylthiochroman-6-yl)-2-propynyl]benzoïque.*

(a) 2'-2'-dibromo-(4,4-diméthylthiochroman-6-yl)ethylène.

Dans un ballon on introduit 5 g (24,2 mmoles) de 4,4-diméthyl-6-thiochroman carboxaldéhyde préparé à l'exemple 11(a) et 50 ml de dichlorométhane. On ajoute successivement 16,1 g (48,4 mmoles) de tétrabromure de carbone 12,7 g (48,4 mmoles) de triphénylphosphine et 3,16 g (48,4 mmoles) de poudre de zinc et agite à température ambiante pendant deux heures. On évapore le milieu réactionnel et purifie le résidu obtenu par chromatographie sur colonne de silice élué avec de l'hexane. On recueille 7,75 g (88%) du produit attendu.

(b) (4,4-diméthylthiochroman-6-yl)acétylène.

Dans un tricol et sous courant d'azote on introduit 7,7 g (21,2 mmoles) de 2'-2'-dibromo-(4,4-diméthylthiochroman-6-yl)ethylène et 80 ml de THF. A -78°C on ajoute goutte à goutte 17 ml (26,6 mmoles) d'une solution de n-butyllithium (2,5 M dans l'hexane) et laisse remonter à température ambiante une heure. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec de l'heptane. On recueille 3,9 g (90%) du dérivé acétylénique attendu sous forme d'une huile jaune.

(c) acide 4-[1-hydroxy-3-(4 4-diméthylthiochroman-6-yl)-2-propynyl]benzoïque.

Dans un tricol et sous courant d'azote on introduit 2 g (9,9 mmoles) de (4,4-diméthylthiochroman-6-yl)acétylène et 25 ml de THF, à -50°C on ajoute goutte à goutte 4 ml (9,9 mmoles) d'une solution de n-butyllithium (2,5 M dans l'hexane) et agite 30'. On ajoute ensuite une solution de 743 mg (4,9 mmoles) de 4-carboxybenzaldéhyde dans 25 ml de THF et agite à température ambiante une heure. On verse le milieu réactionnel dans l'eau, extrait avec de l'éther éthylique, décante la phase organique, sèche sur sulfate de magnésium, évapore. Le résidu est recristallisé dans l'éther diisopropylique, on obtient après filtration 730 mg (42%) de l'acide attendu de point de fusion 168-9°C.

**EXEMPLE 19**

*Acide 2-hydroxy-4-[3-hydroxy-3-(4,4-diméthylthiochroman-6-yl)-1-propynyl]benzoïque.*

De manière analogue à l'exemple 12(b) à partir de 2 g (5,2 mmoles) de 2-hydroxy-4-[3-hydroxy-3-(4,4-diméthylthiochroman-6-yl)-1-propynyl]benzoate de méthyle, on obtient 1,66 g (86%) de l'acide attendu de point de fusion 240-5°C.

**EXEMPLE 20**

*Acide 2-hydroxy-4-[3-hydroxy-3-(4,4-diméthylthiochroman-7-yl)-1-propynyl]benzoïque.*

De manière analogue à l'exemple 12(b) à partir de 2 g (5,2 mmoles) de 2-hydroxy-4-[3-hydroxy-3-(4,4-diméthylthiochroman-7-yl)-1-propynyl]benzoate de méthyle, on obtient 1,55 g (80%) de l'acide attendu de point de fusion 144-5°C.

**EXEMPLE 21**

*Isomère (+) du 4-[3-hydroxy-3-(5,6,7,8-tétrahydro-5,5,8,8-tétraméthyl-2-naphtyl)-1-propynyl]benzoatede méthyle.*

(a) Diastéréoisomère (-) de (R)-α-méthoxyphénylacétate de 1-(5,6,7,8-tétrahydro-5,5,8,8-tétraméthyl-2-naphtyl)-2-propynyle.

Dans un ballon on introduit 9,7 g (40 mmoles) d'α-éthynyl-(5,6,7,8-tétrahydro-5,5,8,8-tétraméthyl-2-naphtalène)méthanol 6,7 g (40 mmoles) d'acide (R)-(-)-α-méthoxyphénylacétique et 100 ml de dichlorométhane. On ajoute successivement 8,3 g (40 mmoles) de dicyclohexylcarbodiimide, 4,9 g (40 mmoles) de 4-diméthylaminopyridine et agite à température ambiante 24 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'heptane (60-40). Après évaporation des solvants, on recueille 3,8 g (97%) de mélange de diastéréoisomères sous forme d'une huile.

La séparation des deux diastéréoisomères est effectuée par deux recristallisations sucessives dans l'isooctane. On obtient ainsi 6 g (38,4%) du diastéréoisomère (-) de point de fusion 94-5°C.

$$\alpha\ ^{20}/_{D} = -14{,}8° \ (c=1,\ CH_2Cl_2)$$

(b) isomère (-) α-éthynyl-(5,6,7,8-tétrahydro-5,5,8,8-tétraméthyl-2-naphtalène) méthanol

Dans un ballon on introduit 5,7 g (14.6 mmoles) du diastéréoisomère (-) préparé précédemment 20 ml de THF et ajoute 10 ml d'une solution de soude méthanolique (2N). On agite à température ambiante pendant une heure, évapore à sec le milieu réactionnel, reprend par l'eau et l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 3,4 g (97%) de l'isomére (-) α-éthynyl-(5,6,7,8-tétrahydro-5,5,8,8-tétraméthyl-2-naphtalène)méthanol de point de fusion 77-8°C.

$$\alpha\ ^{20}/_{D} = -20{,}7° \ (c=1,\ CH_2Cl_2)$$

(c) isomère (+) du 4-[3-hydroxy-3-(5,6,7,8-tétrahydro-5,5,8,8-tétraméthyl-2-naphtyl)-1-propynyl]benzoate de méthyle.

De manière analogue á l'exemple 11(d) par réaction de 1,6 g (6,6 mmoles) de l'isomère (-) α-éthynyl-(5,6,7,8-tétrahydro-5,5,8,8-tétraméthyl-2-naphtalène) méthanol avec 1,7 g (6,6 mmoles) de 4-iodobenzoate de méthyle, on recueille après chromatographie sur colonne de silice élué avec un mélange de dichlorométhane et d'heptane (60-40) 2,1 g (84,6%) de l'isomère (+) du 4-[3-hydroxy-3-(5,6,7,8-tétrahydro-5,5,8,8-tétraméthyl-2-naphtyl)-1-propynyl]benzoate de méthyle de point de fusion 128-9°C.

$$\alpha\ ^{20}/_{D} = +18{,}6° \ (c=1,\ CH_2Cl_2)$$

**EXEMPLE 22**

*Isomère (-) de l'acide 4-[3-hydroxy-3-(5,6,7,8-tétrahydro-5,5,8,8-tétraméthyl-2-naphtyl)-1-propynyl]benzoïque.*

De manière analogue á l'exemple 12 (b) à partir de 1,6g de l'isomère (+) du 4-[3-hydroxy-3-(5,6,7,8-tétrahydro-5,5,8,8-tétraméthyl-2-naphtyl)-1-propynyl] benzoate de méthyle, on obtient 1,1 g (73%) de l'isomère (-) de l'acide 4-(3-hydroxy-3-(5,6,7,8-tétrahydro-5,5,8,8-tétraméthyl-2-naphtyl)-1-propynyl]benzoïque de point de fusion 183-4°C.

$$\alpha\ ^{20}/_{D} = -1{,}1° \ (c=1,\ DMF)$$

**EXEMPLE 23**

*Isomère (+) du 2-hydroxy-4-[3-hydroxy-3-(5,6,7,8-tétrahydro-5,5,8,8-tétraméthyl-2-naphtyl)-1-propynyl]benzoate de méthyle.*

De manière analogue à l'exemple 11(d) par réaction de 1,5 g (6,2 mmoles) de l'isomére (-) α-éthynyl-(5,6,7,8-tétrahydro-5,5,8,8-tétraméthyl-2-naphtalène) méthanol avec 1,7 g (6,2 mmoles) de 2-hydroxy-4-iodobenzoate de méthyle, on recueille après chromatographie sur colonne de silice élué avec un mélange de dichlorométhane et d'heptane (70-30) 2,2 g (91%) de l'isomère (+) du 2-hydroxy-4-[3-hydroxy-3-(5,6,7,8-tétrahydro-5,5,8,8-tétraméthyl-2-naphtyl)-1-propynyl]benzoate de méthyle de point de fusion 100-1°C.

$$\alpha\ ^{20}/_{D} = +17{,}9° \ (c=1,\ CH_2Cl_2)$$

**EXEMPLE 24**

*Isomere (-) de l'acide 2-hydroxy-4-[3-hydroxy-3-(5,6,7,8-tetrahydro-5,5,8,8-tétraméthyl-2-naphtyl)-1-propynyl]benzoique.*

De manière analogue à l'exemple 12 (b) à partir de 1,8 g de l'isomère (+) du 2-hydroxy-4-[3-hydroxy-3-(5,6,7,8-tétrahydro-5,5,8,8-tétraméthyl-2-naphtyl)-1-propynyl]benzoate de méthyle, on obtient 1,5 g (88%) de l'isomère (-) de l'acide 2-hydroxy-4-[3-hydroxy-3-(5,6,7,8-tétrahydro-5,5,8,8-tétraméthyl-2-naphtyl)-1-propynyl]benzoïque de point de fusion 220°C avec décomposition.

$$\alpha\ ^{20}/_{D} = -1° \ (c=1,\ DMF)$$

**EXEMPLE 25**

*Isomère (-) du 4-[3-hydroxy-3-(5,6,7,8-tétrahydro-5,5,8,8-tétraméthyl-2-naphtyl)-1-propynyl]benzoate de méthyle.*

(a) Diastéréoisomère (+) de (S)-α-méthoxyphénylacétate de 1-(5,6,7,8-tétrahydro-5,5,8,8-tétraméthyl-2-naphtyl)-2-propynyle.

Dans un ballon on introduit 9,3 g (38,4 mmoles) d'a-éthynyl-(5,6,7,8-tétrahydro-5,5,8,8-tétraméthyl-2-naphalène)méthanol 6,4 g ( 38,4 mmoles) d'acide (S)-(+)-α-méthoxyphénylacétique et 100 ml de dichlorométhane. On ajoute successivement 7,9 g (38,4 mmoles) de dicyclohexylcarbodiimide. 4,7 g (38,4 mmoles) de 4-diméthylaminopyridine et agite à température ambiante 24 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'heptane (60-40). Après évaporation des solvants, on recueille 12,5 g (84%) de mélange de diastéréoisomères sous forme d'une huile.

La séparation des deux diastéréoisomères est effectuée par deux recristallisations sucessives dans l'isooctane. On obtient ainsi 4 g (27%) du diastéréoisomère (+) de point de fusion 93-4°C.

$$\alpha\ ^{20}/_{D} = -16{,}9° \ (c=1,\ CH_2Cl_2)$$

(b) isomère (+) α-éthynyl-(5,6,7,8-tétrahydro-5,5,8,8-tétraméthyl-2-naphtalène) méthanol

Dans un ballon on introduit 3,7 g (9,5 mmoles) du diastéréoisomère (+) préparé précédemment 20 ml de THF et ajoute 10 ml d'une solution de soude méthanolique (2N). On agite à température ambiante pendant une heure, évapore à sec le milieu réactionnel, reprend par l'eau et l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 1 g (87%) de l'isomère (+) α-éthynyl-(5,6,7,8-tétrahydro-5,5,8,8-tétraméthyl-2-naphtalène)méthanol de point de fusion 77-8°C.

$$\alpha\ ^{20}/_{D} = +18{,}7° \ (c=1,\ CH_2Cl_2)$$

(c) Isomère (-) du 4-[3-hydroxy-3-(5,6,7,8-tétrahydro-5,5,8,8-tétraméthyl-2-naphtyl)-1-propynyl]benzoate de méthyle.

De manière analogue à l'exemple 11(d) par réaction de 700 mg (2,9 mmoles) de l'isomère (+) α-éthynyl-(5,6,7,8-tétrahydro-5,5,8,8-tétraméthyl-2-naphtalène) méthanol avec 760 mg (2,9 mmoles) de 4-iodobenzoate de méthyle, on recueille après chromatographie sur colonne de silice élué avec un mélange de dichlorométhane et d'heptane (60-40) 1 g (92.5%) de l'isomère (-) du 4-[3-hydroxy-3-(5,6,7,8-tétrahydro-5,5,8,8-tétraméthyl-2-naphtyl)-1-propynyl]benzoate de méthyle de point de fusion 128-9°C.

$$\alpha\ ^{20}/_{D} = +18,1° \text{ (c=1, } CH_2Cl_2)$$

**EXEMPLE 26**

*Isomère (-) du 2-hydroxy-4-[3-hydroxy-3-(5,6,7,8-tétrahydro-5,5,8,8-tétraméthyl-2-naphtyl)-1-propynyl]benzoate de méthyle.*

De manière analogue à l'exemple 11(d) par réaction de 1 g (4,1 mmoles) de l'isomère (+) α-éthynyl-(5,6,7,8-tétrahydro-5,5,8,8-tétraméthyl-2-naphtalène) méthanol avec 1,1 g (4,1 mmoles) de 2-hydroxy-4-iodobenzoate de méthyle, on recueille après chromatographie sur colonne de silice élué avec un mélange de dichlorométhane et d'heptane (70-30) 1,45 g (90%) de l'isomére (-) du 2-hydroxy-4-[3-hydroxy-3-(5,6,7,8-tétrahydro-5,5,8,8-tétraméthyl-2-naphtyl)-1- propynyl]benzoate de méthyle de point de fusion 100-1°C.

$$\alpha\ ^{20}/_{D} = -17,6° \text{ (c=1, } CH_2Cl_2)$$

**EXEMPLE 27**

*Isomère (+) de l'acide 4-[3-hydroxy-3-(5,6,7,8-tétrahydro-5,5,8,8-tétraméthyl-2-naphyl)-1-propynyl]benzoïque.*

De manière analogue à l'exemple 12 (b) àpartir de 800 mg de l'isomère (-) du 4-[3-hydroxy-3-(5,6,7,8-tétrahydro-5,5,8,8-tétraméthyl-2-naphtyl)-1-propynyl] benzoate de méthyle, on obtient 600 mg (78%) de l'isomère (+) de l'acide 4-[3-hydroxy-3-(5,6,7,8-tétrahydro-5,5,8,8-tétraméthyl-2-naphtyl)-1-propynyl]benzoïque de point de fusion 180-1°C.

$$\alpha\ ^{20}/_{D} = +1,1° \text{ (c=1, DMF)}$$

**EXEMPLE 28**

*Isomère (+) de l'acide 2-hydroxy-4-[3-hydroxy-3-(5,6,7,8-tétrahydro-5,5,8,8-tétraméthyl-2-naphyl)-1-propynyl]benzoïque.*

De manière analogue à l'exemple 12 (b) à partir de 1.2g de l'isomère (-) du 2-hydroxy-4-[3-hydroxy-3-(5,6,7,8-tétrahydro-5,5,8,8-tétramèthyl-2-naphtyl)-1-propynyl]benzoate de méthyle, on obtient 1 g (87%) de l'isomère (+) de l'acide 2-hydroxy-4-[3-hydroxy-3-(5,6,7,8-tétrahydro-5,5,8,8-tétraméthyl-2-naphtyl)-1-propynyl]benzoïque de point de fusion 220°C avec décomposition.

$$\alpha\ ^{20}/_{D} = +1° \text{ (c=1, DMF)}$$

**EXEMPLE 29**

*2-hydroxy-4-[3-hydroxy-3-méthyl-3-(5,6,7,8-tétrahydro-5,5,8,8-tétraméthyl-2-naphty)-1-propynyl]benzoate de méthyle.*

(a) 1-Triméthylsilyléthynyl-1-(5,6,7,8-tétrahydro-5,5,8,8-tétraméthyl-2-naphtyl) éthanol.

De manière analogue à l'exemple 11(b) par réaction de 5 g (21,7 mmoles) de 2-acétyl-5,6,7,8-tétrahydro-5,5,8,8-tétraméthylnaphtalène avec un équivalent de triméthylsilylacétylénure de lithium, on obtient 6,8 g (95%) d'alcool attendu sous forme d'une huile incolore.

(b) 1-éthynyl-1-(5,6,7,8-tétrahydro-5,5,8,8-tétraméthyl-2-naphtyl)éthanol.

De manière analogue à l'exemple 11(c) à partir de 6,8 g (20,7 mmoles) de 1-Triméthylsilyléthynyl-1-(5,6,7,8-tétrahydro-5,5,8,8-tétraméthyl-2-naphtyl)éthanol, on obtient 4,22 g (75%) de produit attendu de point de fusion 84-5°C.

(c) 2-hydroxy-4-[3-hydroxy-3-méthyl-3-(5,6,7,8-tétrahydro-5,5,8,8-tétraméthyl-2-naphtyl)-1-propynyl]benzoate de méthyle.

De manière analogue à l'exemple 11(d) par réaction de 2 g (7,8 mmoles) de 1-éthynyl-1-(5,6,7,8-tétrahydro-5,5,8,8-tétraméthyl-2-naphtyl)éthanol avec 2,2 g (7,9 mmoles) de 2-hydroxy-4-iodobenzoate de méthyle, on obtient après chromatographie sur colonne de silice élué avec un mélange de dichlorométhane et d'heptane (50-50) 2,77 g (87%) de l'ester attendu de point de fusion 110-5°C.

## EXEMPLE 30

*Acide 2-hydroxy-4-[3-hydroxy-3-méthyl-3-(5,6,7,8-tétrahydro-5,5,8,8-tétraméthyl-2-naphtyl)-1-propynyl]benzoïque.*

De manière analogue à l'exemple 12(b) à partir de 2,2 g (5,4 mmoles) de 2-hydroxy-4-[3-hydroxy-3-méthyl-3-(5,6,7,8-tétrahydro-5,5,8,8-tétraméthyl-2-naphtyl)-1-propynyl]benzoate de méthyle, on obntient 1,9 g (89%) d'acide attendu de point de fusion 265-70°C.

## EXEMPLE 31

*Acide 2-méthoxy-4-[3-hydroxy-3-(5,6,7,8-tétrahydro-5,5,8,8-tétraméthyl-2-naphtyl)-1-propynyl]benzoïque.*

(a) 4-iodo-2-méthoxybenzoate de méthyle.

Dans un tricol on introduit 238 mg (7,9 mmoles) d'hydrure de sodium (80% dans l'huile) et 20 ml de DMF, on ajoute goutte à goutte une solution de 2 g (7,2 mmoles) de 2-hydroxy-4-iodobenzoate de méthyle dans 50 ml de DMF et agite jusqu'à cessation du dégagement gazeux. On ajoute ensuite 540 µl (8,6 mmoles) d'iodométhane et agite à température ambiante deux heures. On verse le milieu réactionnel dans l'eau, extrait avec de l'éther éthylique, décante la phase organique, sèche sur sulfate de magnésium, évapore. On recueille 2,1 g (100%) de 4-iodo-2-méthoxybenzoate de méthyle.

(b) 2-méthoxy-4-[3-hydroxy-3-(5,6,7,8-tétrahydro-5,5,8,8-tétraméthyl-2-naphtyl)-1-propynyl]benzoate de méthyle.

De manière analogue à l'exemple 11(d) par réaction de 1,66 g (6,8 mmoles) de α-éthynyl-(5,6,7,8-tétrahydro-5,5,8,8-tétraméthyl-2-naphtalène)méthanol avec 2 g (6,9 mmoles) de 4-iodo-2-méthoxybenzoate de méthyle, on recueille après chromatographie sur colonne de silice élué avec du dichlorométhane 2,1 g (75%) de l'ester attendu sous forme d'une huile jaune.

(c) Acide 2-méthoxy-4-[3-hydroxy-3-(5,6,7,8-tétrahydro-5,5,8,8-tétraméthyl-2-naphtyl)-1-propynyl]benzoïque.

De manière analogue à l'exemple 12(b) à partir de 2,1 g (5,2 mmoles) de l'ester précédent, on obtient 1 g (50%) de l'acide attendu de point de fusion 100-2°C.

## EXEMPLE 32

*4-[3-hydroxy-3-(5,6,7,8-tétrahydro-5,5,8,8-tétraméthyl-2-naphtyl)-1-propynyl]benzaldéhyde.*

Dans un tricol, on introduit 2,42 g (10 mmoles) d'α-éthynyl-(5,6,7,8-tétrahydro-5,5,8,8-tétraméthyl-2-naphtalène)méthanol, 2 g (11 mmoles) de 4-bromobenzaldéhyde et 50 ml de triéthylamine. On dégaze le milieu réactionnel avec de l'azote pendant 30', puis ajoute sucessivement 169 mg (0,75 mmoles) d'acétate de palladium(II) et 393 mg (1,5 mmoles) de triphénylphosphine. On chauffe à 60°C pendant une heure, évapore à sec le milieu réactionnel, reprend le résidu obtenu par l'eau et l'éther éthylique. On décante la phase organique, sèche sur sulfate de magnésium, évapore. Le résidu obtenu est purifié par chromatographie sur colonne de silice élué avec un mélange de dichlorométhane et d'heptane (70-30), on recueille 1,23 g (35.5%) de 4-[3-hydroxy-3-(5,6,7,8-tétrahydro-5,5,8,8-tétraméthyl-2-naphtyl)-1-propynyl]benzaldéhyde de point de fusion 104-5°C.

**EXEMPLE 33**

*Acétate de 4-[3-hydroxy-3-(5,6,7,8-tétrahydro-5,5,8,8-tétraméthyl-2-naphtyl)-1-propynyl]benzyle.*

De manière analogue à l'exemple 32 par réaction de 2,42 g (10 mmoles) d'a-éthynyl-(5,6,7,8-tétrahydro-5,5,8,8-tétraméthyl-2-naphtalène)méthanol avec 2,52 g (11 mmoles) d'acétate de 4-bromobenzyle, on obtient 790 mg (20%) du produit attendu sous forme d'une huile brune.

**EXEMPLE 34**

*4-[3-hydroxy-3-(5,6,7,8-tétrahydro-5,5,8,8-tétraméthyl-2-naphtyl)-1-propynyl]benzeneméthanol.*

Dans un ballon on introduit 780 mg (2 mmoles) d'acétate de 4-[3-hydroxy-3-(5,6,7,8-tétrahydro-5,5,8,8-tétraméthyl-2-naphtyl)-1-propynyl]benzyle et 30 ml de THF. On ajoute 5 ml d'une solution de soude méthanolique 2N et agite 30', on verse le milieu réactionnel dans l'eau, extrait avec de l'éther éthylique, décante la phase organique, sèche sur sulfate de magnésium, évapore. Le résidu obtenu est trituré dans l'heptane, filtré, on recueille 419 mg (60%) du produit attendu de point de fusion 85-6°C.

**EXEMPLE 35**

*4-[3-hydroxy-3-(5,6,7,8-tétrahydro-5,5,8,8-tétraméthyl-2-naphtyl)-1-propynyl]toluène.*

De manière analogue à l'exemple 11(d) par réaction de 1,2 g (5 mmoles) de α-éthynyl-(5,6,7,8-tétrahydro-5,5,8,8-tétraméthyl-2-naphtalène)méthanol avec 1,1 g (5 mmoles) de 4-iodotoluène, on recueille après chromatographie sur colonne de silice élué avec du dichlorométhane 1,49 g (45%) du produit attendu sous forme d'une huile marron.

**EXEMPLE 36**

*Acetate de 4-[3-hydroxy-3-(5,6,7,8-tétrahydro-3,5,5,8,8-pentaméthyl-2-naphtyl-1-propynyl]phényle.*

(a) 2-bromo-5,6,7,8-tétrahydro-3,5,5,8,8-pentaméthylnaphtalène.

Dans un tricol on introduit 30 ml de 2-bromotoluène et 14 g (0,11 mole) d'$AlCl_3$, on refroidit à 0°C et ajoute goutte à goutte une solution de 50 g (0,27 mole) de 2,5-dichloro-2,5-diméthylhexane dans 100 ml de 2-bromotoluène et laisse remonter à température ambiante. On verse le milieu réactionnel dans l'eau, extrait avec du dichlorométhane, décante la phase organique, lave avec une solution aqueuse de bicarbonate de sodium, évapore. Par agitation dans le méthanol, le produit cristallise, après filtration on recueille 56.9 g (75%) de 2-bromo-5,6,7,8-tétrahydro-3,5,5,8,8-pentaméthylnaphtalène de point de fusion 92-3°C.

(b) 5,6,7,8-tétrahydro-3,5,5,8,8-pentaméthyl-2-naphtalènecarboxaldéhyde.

De manière analogue à l'exemple 11(a) à partir de 8,44 g (30 mmoles) de 2-bromo-5,6,7,8-tétrahydro-3,5,5,8,8-pentaméthylnaphtalène, on obtient 6,9 g (100%) de l'aldéhyde attendu de point de fusion 75-6°C.

(c) α-Triméthylsilyléthynyl-(5,6,7,8-tétrahydro-3,5,5,8,8-pentaméthyl-2-naphtalène)méthanol.

De manière analogue à l'exemple 11(b) par réaction de 6,66 g (29 mmoles) de 5,6,7,8-tétrahydro-3,5,5,8,8-pentaméthyl-2-naphtalènecarboxaldéhyde avec un équivalent de triméthylsilylacétylénure de lithium, on obtient 8,8 g (92%) de l'alcool attendu de point de fusion 95-6°C.

(d) α-éthynyl-(5,6,7,8-tétrahydro-3,5,5,8,8-pentaméthyl-2-naphtalène)méthanol.

De manière analogue à l'exemple 11(c) à partir de 8,6 g (26 mmoles) de α-Triméthylsilyléthynyl-(5,6,7,8-tétrahydro-3,5,5,8,8-pentaméthyl-2-naphtalène)méthanol, on obtient 4,8 g (72%) de produit attendu de point de fusion 101-2°C.

(e) Acétate de 4-[3-hydroxy-3-(5,6,7,8-tétrahydro-3,5,5,8,8-pentaméthyl-2-naphtyl)-1-propynyl]phényle.

De manière analogue à l'exemple 11(d) par réaction de 1.28 g (5 mmoles) de α-éthynyl-(5,6,7,8-tétrahydro-3,5,5,8,8-pentaméthyl-2-naphtalène)méthanol avec 1,44 g (5,5 mmoles) d'acétate de 4-iodophényle, on obtient 480 mg (25%) du produit attendu de point de fusion 129-30°C.

**EXEMPLE 37**

*4-[3-hydroxy-3-(5,6,7,8-tétrahydro-3,5,5,8,8-pentaméthyl-2-naphtyl)-1-propynyl]phénol.*

De manière analogue à l'exemple 34 à partir de 300 mg (0,77 mmole) d'acétate de 4-[3-hydroxy-3-(5,6,7,8-tétrahydro-3,5,5,8,8-pentaméthyl-2-naphtyl)-1-propynyl]phényle, on obtient 207 mg (77%) de 4-[3-hydroxy-3-(5,6,7,8-tétrahydro-3,5,5,8,8-pentaméthyl-2-naphtyl)-1-propynyl]phénol de point de fusion 158-9°C.

**EXEMPLE 38**

*4-[3-hydroxy-3-(5,6,7,8-tétrahydro-5,5,8,8-tétraméthyl-2-naphtyl)-1-propynyl]phénylsulfinylméthane.*

(a) 4-bromophénylsulfinylméthane.

Dans un ballon on introduit 4,06 g (20 mmoles) de 4-bromothioanisole et 75 ml de dichlorométhane et ajoute 6,3 g (20 mmoles) d'acide méta-chloro perbenzoïque. On agite à température ambiante pendant quatre heures, verse le milieu réactionnel dans l'eau, décante la phase organique, sèche sur sulfate de magnésium, évapore. Le résidu obtenu est purifié par chromatographie sur colonne de silice élué avec un mélange de dichlorométhane et d'heptane (70-30). Après évaporation des solvants on recueille 1,22 g (28%) du sulfoxyde attendu de point de fusion 80-1°C.

(b) 4-[3-hydroxy-3-(5,6,7,8-tétrahydro-5,5,8,8-tétraméthyl-2-naphtyl)-1-propynyl]phénylsulfinylméthane.

De manière analogue à l'exemple 32 par réaction de 1,21 g (5 mmoles) d'a-éthynyl-(5,6,7,8-tétrahydro-5,5,8,8-tétraméthyl-2-naphtalène)méthanol avec 1,1 g (5 mmoles) de 4-bromophénylsulfinylméthane, on obtient 177 mg (9%) du dérivé sulfoxyde attendu de point de fusion 121-2°C.

**EXEMPLE 39**

*4-[3-hydroxy-3-(5,6,7,8-tétrahydro-5,5,8,8-tétraméthyl-2-naphtyl)-1-propynyl]phénylsulfonylméthane.*

(a) 4-bromophénylsulfonylméthane.

De manière analogue à l'exemple 38(a) par réaction de 2,03 g (10 mmoles) de 4-bromothioanisole avec 10,35 g (30 mmoles) d'acide métachloroperbenzoïque, on obtient 1,72 g (73%) de la sulfone attendu de point de fusion 94-5°C.

(b) 4-[3-hydroxy-3-(5,6,7,8-tétrahydro-5,5,8,8-tétraméthyl-2-naphtyl)-1-propynyl]phénylsulfonylméthane.

De manière analogue à l'exemple 32 par réaction de 1,21 g (5 mmoles) d'a-éthynyl-(5,6,7,8-tétrahydro-5,5,8,8-tétraméthyl-2-naphtalène)méthanol avec 1,18 g (5 mmoles) de 4-bromophénylsulfinylméthane, on obtient 610 mg (31%) du dérivé sulfone attendu de point de fusion 112-3°C.

**EXEMPLE 40**

*N-éthyl-4-[3-hydroxy-3-(5,6,7,8-tétrahydro-5,5,8,8-tétraméthyl-2-naphtyl)-1-propynyl]benzamide.*

(a) chlorure de 4-iodobenzoyle.

Dans un ballon on introduit 5 g (20 mmoles) d'acide 4-iodobenzoïque 30 ml de toluène et 5 gouttes de DMF. On chauffe à 40°C, ajoute 1,74 ml (24 mmoles) de chlorure de thionyle et agite pendant trente minutes. On évapore à sec et recueille 5,5 g (100%) du chlorure d'acide brut qui sera utilisé tel quel pour la suite de la synthèse.

b) N-éthyl-4-iodobenzamide.

Dans un ballon on introduit 90 ml (45 mmoles) d'une solution 0,5N d'éthylamine dans le THF et ajoute goutte à goutte une solution de 4 g (15 mmoles) de chlorure de 4-iodobenzoyle dans 20 ml de dichlorométhane. On agite à température ambiante une heure, verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec du dichlorométhane. Après évaporation des solvants on recueille 3,41 g (82%) de l'amide attendu.

c) N-éthyl-4-[3-hydroxy-3-(5,6,7,8-tétrahydro-5,5,8,8-tétraméthyl-2-naphtyl)-1-propynyl]benzamide.

De manière analogue à l'exemple 11(d) par réaction de 1,21 g (5 mmoles) de α-éthynyl-(5,6,7,8-tétrahydro-3,5,5,8,8-pentaméthyl-2-naphtalène)méthanol avec 1,37 g (5,1 mmoles) de N-éthyl-4-iodobenzamide, on obtient 596 mg (31%) de l'amide attendu de point de fusion 153-4°C.

**EXEMPLE 41**

*N,N'-diéthyl-4-[3-hydroxy-3-(5,6,7,8-tétrahydro-5,5,8,8-tétraméthyl-2-naphtyl)-1-propynyl]benzamide.*

a) N,N'-diéthyl-4-iodobenzamide.

De manière analogue à l'exemple 40(b) par réaction de 5 g (18 mmoles) de chlorure de 4-iodobenzoyle avec 5,6 ml (54 mmoles) de diéthylamine, on obtient 3,4 g (62%) de l'amide attendu.

(b) N,N'-diéthyl-4-[3-hydroxy-3-(5,6,7,8-tétrahydro-5,5,8,8-tétraméthyl-2-naphtyl)-1-propynyl]benzamide.

De manière analogue à l'exemple 11(d) par réaction de 970 mg (4 mmoles) de α-éthynyl-(5,6,7,8-tétrahydro-3,5,5,8,8-pentaméthyl-2-naphtalène)méthanol avec 1,25 g (4,1 mmoles) de N,N'-diéthyl-4-iodobenzamide, on obtient 584 mg (35%) de l'amide attendu sous forme d'une huile marron.

**EXEMPLE 42**

*Morpholide de l'acide 4-[3-hydroxy-3-(5,6,7,8-tétrahydro-5,5,8,8-tétraméthyl-2-naphtyl)-1-propynyl]benzoïque.*

(a) Morpholide de l'acide 4-iodobenzoïque.

De manière analogue à l'exemple 40(b) par réaction de 4 g (15 mmoles) de chlorure de 4-iodobenzoyle avec 3,9 ml (45 mmoles) de morpholine, on obtient 3,64 g (76%) de l'amide attendu.

(b) Morpholide de l'acide 4-[3-hydroxy-3-(5,6,7,8-tétrahydro-5,5,8,8-tétraméthyl-2-naphtyl)-1-propynyl]benzoïque.

De manière analogue à l'exemple 11(d) par réaction de 1,21 g (5 mmoles) de α-éthynyl-(5,6,7,8-tétrahydro-3,5,5,8,8-pentaméthyl-2-naphtalène)méthanol avec 1,62 g (5,1 mmoles) de Morpholide de l'acide 4-iodobenzoïque, on obtient 423 mg (20%) de l'amide attendu de point de fusion 122-3°C.

**EXEMPLE 43**

*5-[3-hydroxy-3-(5,6,7,8-tétrahydro-5,5,8,8-tétraméthyl-2-naphtyl)-1-propynyl]2-thiophènecarboxylate de méthyle.*

De manière analogue à l'exemple 32 par réaction de 1,21 g (5 mmoles) d'α-éthynyl-(5,6,7,8-tétrahydro-5,5,8,8-tétraméthyl-2-naphtalène)méthanol avec 1,1 g (5 mmoles) de 5-bromo-2-thiophènecarboxylate de méthyle, on obtient 371 mg (19%) de l'ester méthylique attendu de point de fusion 84-5°C.

**EXEMPLE 44**

*2-hydroxy-4-[3-hydroxy-3-(5,6,7,8-tétrahydro-3,5,5,8,8-pentaméthyl-2-naphtyl)-1-propynyl]benzoate de méthyle.*

De manière analogue à l'exemple 11(d) par réaction de 1,28 g (5 mmoles) de α-éthynyl-(5,6,7,8-tétrahydro-3,5,5,8,8-pentaméthyl-2-naphtalène)méthanol [préparé à l'exemple 37 (d)] avec 1,5 g (5,5 mmoles) de 2-hydroxy-4-iodobenzoate de méthyle, on obtient 739 mg (36%) du produit attendu de point de fusion 112-3°C.

**EXEMPLE 45**

*Acide 2-hydroxy-4-[3-hydroxy-3-(5,6,7,8-tétrahydro-3,5,5,8,8-pentaméthyl-2-naphtyl)-1-propynyl]benzoïque.*

De manière analogue à l'exemple 12(b) à partir de 600 mg (1,5 mmole) de 2-hydroxy-4-[3-hydroxy-3-(5,6,7,8-tétrahydro-3,5,5,8,8-pentaméthyl-2-naphtyl)-1-propynyl]benzoate de méthyle, on obtient 132 mg (23%) de l'acide attendu de point de fusion 88-9°C.

**EXEMPLE 46**

*2-hydroxy-4-[3-hydroxy-3-(3-méthoxy-5,6,7,8-tétrahydro-5,5,8,8-tétraméthyl-2-naphtyl)-1-propynyl]benzoate de méthyle.*

(a) 2-bromo-3-hydroxy-5,6,7,8-tétrahydro-5,5,8,8-tétraméthylnaphtalène.

Dans un tricol on introduit successivement 36,6 g (0,2 mole) de 2,5-dichloro-2,5-diméthylhexane 34,6 g (0,2 mole) de 2-bromophénol et 400 ml de dichlorométhane. A 0°C on ajoute par petites quantités 26,6 g (0,2 mole) d'$AlCl_3$ et agite jusqu'à cessation du dégagement geazeux (réaction violente). On verse le milieu réactionnel dans l'eau, décante la phase organique, lave avec une solution aqueuse de bicarbonate de sodium, sèche sur sulfate de magnésium, évapore. Le résidu est purifié par chromatographie sur colonne de silice élué avec un mélange d'acétate d'éthyle et d'heptane (10-90). Après évaporation des solvants, on recueille 20,6 g (36%) du phénol attendu.

(b) 2-bromo-3-méthoxy-5,6,7,8-tétrahydro-5,5,8,8-tétraméthylnaphtalène.

Dans un tricol on introduit 720 mg (24 mmoles) d'hydrure de sodium (80% dans l'huile) et 20 ml de DMF, on ajoute goutte à goutte une solution de 5,7 g (20 mmoles) de 2-bromo-3-hydroxy-5,6,7,8-tétrahydro-5,5,8,8-tétraméthylnaphtalène dans 75 ml de DMF et agite jusqu'à cessation du dégagement gazeux. On ajoute ensuite 1,5 ml (24 mmoles) d'iodométhane et agite à température ambiante deux heures. On verse le milieu réactionnel dans l'eau, extrait avec de l'éther éthylique, décante la phase organique, sèche sur sulfate de magnésium, évapore. Le résidu obtenu est trituré dans l'heptane, filtré. On recueille 5,5 g (93%) de 2-bromo-3-méthoxy-5,6,7,8-tétrahydro-5,5,8,8-tétraméthylnaphtalène de point de fusion 70-1°C.

(b) 3-méthoxy-5,6,7,8-tétrahydro-5,5,8,8-tétraméthyl-2-naphtalènecarboxaldéhyde.

De manière analogue à l'exemple 11(a) à partir de 5,3 g (17,8 mmoles) de 2-bromo-3-méthoxy-5,6,7,8-tétrahydro-5,5,8,8-tétraméthylnaphtalène, on obtient 3,5 g (80%) de l'aldéhyde attendu de point de fusion 125-6°C.

(c) α-Triméthylsilyléthynyl-(3-méthoxy-5,6,7,8-tétrahydro-5,5,8,8-tétraméthyl-2-naphtalène)méthanol.

De manière analogue à l'exemple 11(b) par réaction de 3,21 g (13 mmoles) de 3-méthoxy-5,6,7,8-tétrahydro-5,5,8,8-tétraméthyl-2-naphtalènecarboxaldéhyde avec un équivalent de triméthylsilylacétylénure de lithium, on obtient 4,4 g (99%) de l'alcool attendu sous forme d'une huile jaune.

(d) α-éthynyl-(3-méthoxy-5,6,7,8-tétrahydro-5,5,8,8-tétraméthyl-2-naphtalène)méthanol.

De manière analogue à l'exemple 11(c) à partir de 4,4 g (12,7 mmoles) de α-triméthylsilyléthynyl-(3-méthoxy-5,6,7,8-tétrahydro-5,5,8,8-tétraméthyl-2-naphtalène)méthanol, on obtient 1,15 g (33%) de produit attendu

(e) 2-hydroxy-4-[3-hydroxy-3-(3-méthoxy-5,6,7,8-tétrahydro-5,5,8,8-tétraméthyl-2-naphtyl)-1-propynyl]benzoate de méthyle.

De manière analogue à l'exemple 11(d) par réaction de 1,15 g (4,2 mmoles) de α-éthynyl-(3-méthoxy-5,6,7,8-tétrahydro-5,5,8,8-tétraméthyl-2-naphtalène)méthanol avec 1,21 g (4,64 mmoles) de 2-hydroxy-4-iodobenzoate de méthyle, on obtient 1,36 g (76%) du produit attendu de point de fusion 125-6°C.

**EXEMPLE 47**

*Acide 2-hydroxy-4-[3-hydroxy-3-(3-méthoxy-5,6,7,8-tétrahydro-5,5,8,8-tétraméthyl-2-naphtyl)-1-propynyl]benzoïque.*

De manière analogue à l'exemple 12(b) à partir de 1,1 g (2,6 mmoles)de 2-hydroxy-4-[3-hydroxy-3-(3-méthoxy-5,6,7,8-tétrahydro-5,5,8,8-tétraméthyl-2-naphtyl)-1-propynyl]benzoate de méthyle, on obtient 890 mg (84%) de l'acide attendu de point de fusion 225-8°C.

**EXEMPLE 48**

*Acide 4-[3-hydroxy-3-(4,4-diméthylthiochroman-6-yl)-1-propynyl]benzoïque.*

(a) 4-[3-hydroxy-3-(4,4-diméthylthiochroman-6-yl)-1-propynyl]benzoate de méthyle.

De manière analogue à l'exemple 11(d) par réaction de 1,4 g (6 mmoles) d'α-éthynyl-(4,4-diméthyl-6-thiochroman)méthanol avec 1,6 g (6 mmoles) de 4-iodobenzoate de méthyle, on obtient 1,5 g (68%) de l'ester attendu sous forme d'une huile orangée.

(b) acide 4-[3-hydroxy-3-(4,4-diméthylthiochroman-6-yl)-1-propynyl]benzoïque.

De manière analogue à l'exemple 12(b) à partir de 1 g (2,7 mmoles) de 4-[3-hydroxy-3-(4,4-diméthylthiochroman-6-yl)-1-propynyl]benzoate de méthyle, on obtient 450 mg (47%) de l'acide attendu de point de fusion 147-9°C.

**EXEMPLE 49**

*Acide 4-[3-hydroxy-3-(4,4-diméthylthiochroman-7-yl)-1-propynyl]benzoïque.*

(a) 4-[3-hydroxy-3-(4,4-diméthylthiochroman-7-yl)-1-propynyl]benzoate de méthyle.

De manière analogue à l'exemple 11(d) par réaction de 2 g (8,6 mmoles) d'α-éthynyl-(4,4-diméthyl-7-thiochroman)méthanol avec 2,25 g (8,6 mmoles) de 4-iodobenzoate de méthyle, on obtient 1,8 g (57%) de l'ester attendu sous forme d'une huile jaune.

(b) acide 4-[3-hydroxy-3-(4,4-diméthylthiochroman-7-yl)-1-propynyl]benzoïque.

De manière analogue à l'exemple 12(b) à partir de 1,2 g (3,3 mmoles) de 4-[3-hydroxy-3-(4,4-diméthylthiochroman-7-yl)-1-propynyl]benzoate de méthyle, on obtient 300 mg (26%) de l'acide attendu de point de fusion 151-3°C.

**EXEMPLE 50**

*3-méthyl-4-[3-hydroxy-3-(5,6,7,8-tétrahydro-5,5,8,8-tétraméthyl-2-naphtyl)-1-propynyl]benzoate de méthyle.*

(a) acide 3-méthyl-4-iodobenzoïque.

Dans un tricol on introduit 20 g (0,132 mole) d'acide 3-méthyl-4-amino benzoïque 175 ml d'acide sulfurique (20%). A -10°C on ajoute goutte à goutte une solution de 11,9 g (0,172 mole) de nitrite de sodium dans 50 ml d'eau et agite pendant deux heures. Cette solution est introduite goutte à goutte par l'intermédiaire d'une ampoule réfrigérée à -5°C dans une solution de 35 g (0,211 mole) d'iodure de potassium 35,2 g (0,185 mole) d'iodure de cuivre et 175 ml d'acide sulfurique (20%). On agite pendant huit heures, filtre le milieu réactionnel, dissout le solide obtenu dans l'acétate d'éthyle, lave à l'eau puis avec une solution de sulfite de sodium,sèche sur sulfate de magnésium, évapore. On recueille 24,4 g (70%) d'acide 3-méthyl-4-iodobenzoïque de point de fusion 205-10°C.

(b) 3-méthyl-4-iodobenzoate de méthyle.

Dans un ballon on introduit 24,4 g (0,093 mole) d'acide 3-méthyl-4-iodobenzoïque 250 ml de méthanol et ajoute goutte à goutte 2.5 ml d'acide sulfurique concentré. On chauffe à reflux pendant douze heures, évapore le milieu réactionnel, reprend avec acétate d'éthyle et eau, décante la phase organique, sèche sur sulfate de magnésium, évapore. Le résidu est trituré dans le méthanol, filtré, on recueille 21,9 g (85%) de l'ester méthylique attendu de point de fusion 58-9°C.

(c) 3-méthyl-4-[3-hydroxy-3-(5,6,7,8-tétrahydro-5,5,8,8-tétraméthyl-2-naphtyl)-1-propynyl]benzoate de méthyle.

De manière analogue à l'exemple 11(d) par réaction de 2,4 g (10 mmoles) d'α-éthynyl-(5,6,7,8-tétrahydro-5,5,8,8-tetraméthyl-2-naphtaléne)méthanol avec 2,7 g (10 mmoles) de 3-méthyl-4-iodobenzoate de méthyle, on obtient 3,2 g (83%) de l'ester attendu de point de fusion 130-1°C.

**EXEMPLE 51**

*Acide 3-méthyl-4-[3-hydroxy-3-(5,6,7,8-tétrahydro-5,5,8,8-tétraméthyl-2-naphtyl)-1-propynyl]benzoïque.*

De manière analogue à l'exemple 12(b) à partir de 2,2 g (5,6 mmoles) de 3-méthyl-4-[3-hydroxy-3-(5,6,7,8-tétrahydro-5,5,8,8-tétraméthyl-2-naphtyl)-1-propynyl]benzoate de méthyle, on obtient 1,5 g (71%) d'acide 3-méthy-4-[3-hydroxy-3-(5,6,7,8-tétrahydro-5,5,8,8-tétraméthyl-2-naphtyl)-1-propynyl]benzoïque de point de fusion 189-90°C.

**EXEMPLE 52**

*Acide 2-chloro-4-[3-hydroxy-3-(5,6,7,8-tétrahydro-5,5,8,8-tétraméthyl-2-naphtyl)-1-propynyl]benzoïque.*

(a) acide 2-chloro-4-iodobenzoïque.

De manière analogue à l'exemple 50(a) à partir de 10 g (58,3 mmoles) d'acide 2-chloro-4-aminobenzoïque, on recueille 14,26 g (86%) d'acide 2-chloro-4-iodo benzoïque.

(b) 2-chloro-4-iodobenzoate de méthyle.

De manière analogue à l'exemple 50(b) à partir de 13,9 g (49,2 mmoles) d'acide 2-chloro-4-iodobenzoïque, on obtient 11,52 g (79%) de l'ester méthylique attendu sous forme d'une huile.

(c) 2-chloro-4-[3-hydroxy-3-(5,6,7,8-tétrahydro-5,5,8,8-tétraméthyl-2-naphtyl)-1-propynyl]benzoate de méthyle.

De maniére anlogue à l'exemple 11(d) par réaction de 1,2 g (5 mmoles) d'α-éthynyl-(5,6,7,8-tétrahydro-5,5,8,8-tetraméthyl-2-naphtalène)méthanol avec 1,6 g (5 mmoles) de 2-chloro-4-iodobenzoate de méthyle, on obtient 1,7 g (83%) de l'ester attendu sous forme d'une huile marron.

(d) acide 2-chloro-4-[3-hydroxy-3-(5,6,7,8-tétrahydro-5,5,8,8-tétraméthyl-2-naphtyl)-1-propynyl]benzoïque.

De manière analogue à l'exemple 12(b) à partir de 1,7 g (4,1 mmoles) de 2-chloro-4-[3-hydroxy-3-(5,6,7,8-tétrahydro-5,5,8,8-tétraméthyl-2-naphtyl)-1-propynyl]benzoate de méthyle, on obtient 730 mg (44%) d'acide 2-chloro-4-[3-hydroxy-3-(5,6,7,8-tétrahydro-5,5,8,8-tétraméthyl-2-naphtyl)-1-propynyl]benzoïque de point de fusion 145-8°C.

**EXEMPLE 53**

*Acide 2-acétoxy-4-[3-acétoxy-3-(5,6,7,8-tétrahydro-5,5,8,8-tétraméthyl-2-naphtyl)-1-propynyl]benzoïque.*

Dans un ballon, on introduit 500 mg (1,3 mmoles) d'acide 2-hydroxy-4-[3-hydroxy-3-(5,6,7,8-tétrahydro-5,5,8,8-tétraméthyl-2-naphtyl)-1-propynyl]benzoïque 10 ml de pyridine et ajoute goutte à goutte 150 µL (1,56 mmoles) d'anhydride acétique. On agite à température ambiante pendant 2 heures, évapore à sec le milieu réactionnel, reprend le résidu par l'eau, acidifie à pH 4, extrait avec de l'éther éthylique, sèche sur sulfate de magnésium, évapore. Le résidu obtenu est purifié par chromatographie sur colonne de silice élué avec de l'éther éthylique, on recueille 230 mg (39%) d' acide 2-acétoxy-4-[3-acétoxy-3-(5,6,7,8-tétrahydro-5,5,8,8-tétraméthyl-2-naphtyl)-1-propynyl] benzoïque de point de fusion 113-5°C.

**EXEMPLE 54**

*4-[3-hydroxy-3-(3-tert-butyl-4-propyloxyphényl)-1-propynyl]benzoate de méthyle.*

(a) 3-tert-butyl-4-propyloxybromobenzène.

De manière analogue à l'exemple 46(b) par réaction de 4,58 g (0,02 mole) de 3-tert-butyl-4-hydroxybromobenzène avec 2,2 ml (0,022 mole) de 1-iodopropane, on obtient 4,7 g (87%) de 3-tert-butyl-4-propyloxybromobenzène sous forme d'une huile incolore.

(b) 3-tert-butyl-4-propyloxybenzaldéhyde.

De manière analogue à l'exemple 11(a) à partir de 4,5 g (16,6 mmoles) de 3-tert-butyl-4-propyloxybromobenzène, on obtient 3,65 g (100%) de l'aldéhyde attendu sous forme d'une huile légèrement jaune.

(c) α-Triméthylsilyléthynyl-(3-tert-butyl-4-propyloxybenzène)méthanol.

De manière analogue à l'exemple 11(b) par réaction de 3,6 g (16,4 mmoles) de 3-tert-butyl-4-propyloxybenzaldéhyde avec un équivalent de triméthylsilylacétylénure de lithium, on obtient 5,2 g (100%) d'alcool attendu sous forme d'une huile incolore.

(d) α-éthynyl-(3-tert-butyl-4-propyloxybenzène)méthanol.

De manière analogue à l'exemple 11(c) à partir de 5,1 g (14,1 mmoles) d'α-Triméthylsilyléthynyl-(3-tert-butyl-4-propyloxybenzène)méthanol, on obtient après chromatographie sur colonne de silice élué avec un mélange de dichlorométhane et d'heptane (60-40) 3,3 g (80%) d' α-éthynyl-(3-tert-butyl-4-propyloxybenzène) méthanol sous forme d'une huile jaune.

(e) 4-[3-hydroxy-3-(3-tert-butyl-4-propyloxyphényl)-1-propynyl]benzoate de méthyle.

De manière analogue à l'exemple 11(d) par réaction de 3,3 g (13,4 mmoles) d'α-éthynyl-(3-tert-butyl-4-propyloxybenzène)méthanol avec 3,8 g (13,4 mmoles) de 2-hydroxy-4-iodobenzoate de méthyle, on obtient 4,4 g (83%) de 4-[3-hydroxy-3-(3-tert-butyl-4-propyloxyphényl)-1-propynyl]benzoate de méthyle sous forme d'une huile marron foncé.

**EXEMPLE 55**

*4-[3-hydroxy-3-(3-tert-butyl-4-héxyloxyphényl)-1-propynyl]benzoate de méthyle*

a) 3-tert-butyl-4-héxyloxybromobenzène.

De manière analogue à l'exemple 46(b) par réaction de 4,58 g (0,02 mole) de 3-tert-butyl-4-hydroxybromobenzène avec 3,3 ml (0,022 mole) de 1-iodohexane, on obtient 6 g (97%) de 3-tert-butyl-4-héxyloxybromobenzène sous forme d'une huile incolore.

(b) 3-tert-butyl-4-héxyloxybenzaldéhyde.

De manière analogue à l'exemple 11(a) à partir de 5,9 g (18,8 mmoles) de 3-tert-butyl-4-héxyloxybromobenzène, oin obtient 4,9 g (100%) de l'aldéhyde attendu sous forme d'une huile légèrement jaune.

(c) α-Triméthylsilyléthynyl-(3-tert-butyl-4-héxyloxybenzène)méthanol.

De manière analogue à l'exemple 11(b) par réaction de 4,8 g (18,3 mmoles) de 3-tert-butyl-4-héxyloxybenzaldéhyde avec un équivalent de triméthylsilylacétylénure de lithium, on obtient 6,6 g (100%) d'alcool attendu sous forme d'une huile incolore.

d) α-éthynyl-(3-tert-butyl-4-héxyloxybenzène)méthanol.

De manière analogue à l'exemple 11(c) à partir de 6,6 g (18,3 mmoles) d'α-Triméthylsilyléthynyl-(3-tert-butyl-4-hexyloxybenzène)méthanol, on obtient après chromatographie sur colonne de silice élué avec un mélange de dichlorométhane et d'heptane (60-40) 4,3 g (81%) d' α-éthynyl-(3-tert-butyl-4-héxyloxybenzène)méthanol sous forme d'une huile jaune.

(e) 4-[3-hydroxy-3-(3-tert-butyl-4-héxyloxyphényl)-1-propynyl]benzoate de méthyle.

De manière analogue à l'exemple 11(d) par réaction de 3,8 g (13,2 mmoles) d'α-éthynyl-(3-tert-butyl-4-héxyloxybenzène)méthanol avec 3,7 g (13,7 mmoles) de 2-hydroxy-4-iodobenzoate de méthyle, on obtient4 g (69%) de 4-[3-hydroxy-3-(3-tert-butyl-4-héxyloxyphényl)-1-propynyl]benzoate de méthyle sous forme d'une huile marron foncé.

**EXEMPLE 56**

Dans cet exemple, on a illustré diverses formulations concrètes à base des composés selon l'invention.

A- VOIE ORALE

| (a) Comprimé de 0,2 g | |
|---|---|
| - Composé préparé à l'exemple 7 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |
| (b) Suspension buvable en ampoules de 5 ml | |
| - Composé préparé à l'exemple 3 | 0,001 g |
| - Glycérine | 0,500 g |
| - Sorbitol à 70% | 0,500 g |
| - Saccharinate de sodium | 0,010 g |
| - Parahydroxybenzoate de méthyle | 0,040 g |
| - Arome qs | |
| - Eau purifiée qsp | 5 ml |
| (c) Comprimé de 0,8 g | |
| - Composé de l'exemple 6 | 0,500 g |
| - Amidon prégélatinisé | 0,100 g |
| - Cellulose microcristalline | 0,115 g |
| - Lactose | 0,075 g |
| - Stéarate de magnésium | 0,010 g |
| (d) Suspension buvable en ampoules de 10 ml | |
| - Composé de l'exemple 2 | 0,200 g |
| - Glycérine | 1,000g |
| - Sorbitol à 70% | 1,000g |
| - Saccharinate de sodium | 0,010 g |
| - Parahydroxybenzoate de méthyle | 0,080 g |
| - Arome qs | |
| - Eau purifiée qsp | 10 ml |

B- VOIE TOPIQUE

| | |
|---|---|
| (a) Onguent | |
| - Composé de l'exemple 9 | 0,020 g |
| - Myristate d'isopropyle | 81,700 g |
| - Huile de vaseline fluide | 9,100 g |
| - Silice ("Aérosil 200" vendue par DEGUSSA) | 9,180 g |
| (b) Onguent | |
| - Composé de l'exemple 10 | 0,300 g |
| - Vaseline blanche codex | 100 g |
| (c) Crème Eau-dans-Huile non ionique | |
| - Acide 2-hydroxy-4-[3-hydroxy-3-(3-tert-butyl-4-hydroxyphényl)]-1-propynylbenzoïque | 0,100 g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |
| (d) Lotion | |
| - Composé de l'exemple 8 | 0,100 g |
| - Polyéthylène glycol (PEG 400) | 69,900 g |
| - Ethanol à 95% | 30,000 g |
| (e) Onguent hydrophobe | |
| - Composé de l'exemple 7 | 0,300 g |
| - Mirystate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) | 100g |
| (f) Crème Huile-dans-Eau non ionique | |
| - Composé de l'exemple 3 | 1,000 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile | 100 g |

**Revendications**

1. Composés propynyl bi-aromatiques, caractérisés par le fait qu'ils répondent à la formule générale (I) suivante :

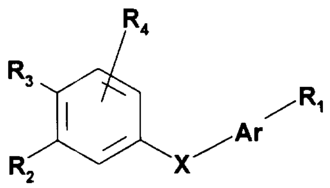

(I)

dans laquelle :

* Ar représente un radical choisi parmi les radicaux de formules (a)-(e) suivantes :

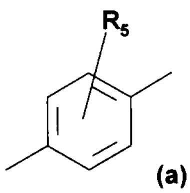 (a)

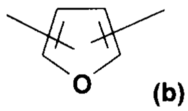 (b)

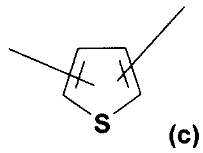 (c)

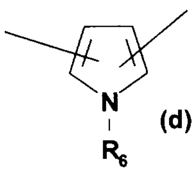 (d)

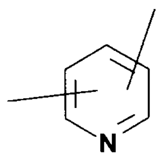 (e)

$R_5$ et $R_6$ ayant la signification donnée ci-après,

* $R_1$ représente :

(i) un atome d'hydrogène

(ii) un radical $-CH_3$

(iii) un radical $-CH_2-O-R_6$

(iv) un radical $-O-R_6$

(v) un radical $-CO-R_7$,

(vi) un radical $-S(O)_tR_9$

$R_6$, $R_7$, $R_9$ et t ayant la signification donnée ci-après,

* X représente un radical de formule :

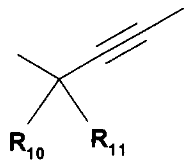

ou

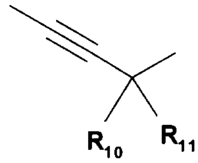

$R_{10}$ et $R_{11}$ ayant la signification donnée ci-après,

* $R_2$ et $R_3$ représentent un atome d'hydrogène, un radical alkyle linéaire ou ramifié ayant de 1 à 20 atomes de carbone, un radical $-OR_6$ ou un radical $-SR_6$, $R_6$ ayant la signification donnée ci-après, étant entendu que $R_2$ et $R_3$, pris ensemble, peuvent former avec le cycle aromatique adjacent un cycle à 5 ou 6 chainons éventuellement substitué par des groupes méthyle et/ou éventuellement interrompu par un atome d'oxygène ou de soufre,

* $R_4$ représente un atome d'hydrogène, un atome d'halogène, un radical alkyle en C1-C6 ou un radical $-OR_6$, $R_6$ ayant la signification donnée ci-après,

étant entendu que dans tout ce qui précède :

- $R_5$ a la même signification que $R_4$,

- $R_6$ représente un atome d'hydrogène, un radical alkyle en C1-C6 ou un radical $-CO-R_9$, $R_9$ ayant la signification donnée ci-après,

- $R_7$ représente :

  (a) un atome d'hydrogène
  (b) un radical alkyle en C1-C6
  (c) un radical de formule :

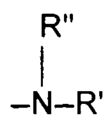

  R' et R" ayant la signification donnée ci-après,
  (d) un radical $-OR_8$, $R_8$ ayant la signification donnée ci-après,

- $R_8$ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,

- $R_9$ représente un radical alkyle en C1-C6,

- $R_{10}$ représente un atome d'hydrogène, un radical alkyle en C1-C6 ou un radical $-OR_6$,

- $R_{11}$ représente un radical $-OR_6$,

- R' et R" représentent un atome d'hydrogène, un radical alkyle en C1-C6, un radical mono ou polyhydroxyalkyle, un radical aryle éventuellement substitué ou un reste d'aminoacide ou de peptide ou de sucre ou encore, pris ensemble, forment un hétérocycle,

- t est un nombre entier égal à 0, 1 ou 2,

- les radicaux $R_{10}$ et $R_{11}$ ci-dessus, pris ensemble, peuvent former un radical oxo unique de formule =O, avec les conditions suivantes :

  - si R10 représente un atome d'hydrogène, R11 un radical hydroxyle, et R2 et R3 ne forment pas avec le cycle aromatique adjacent un cycle à 5 ou 6 chainons, alors R4 ne représente pas un radical hydroxyle, quand R4 est en position ortho de la position $\alpha$ du cycle;
  - si R10 représente un atome d'hydrogène et R11 un radical hydroxyle, ou R10 et R11 forment un radical oxo unique de formule =O, et R2 et R3 ne forment pas avec le cycle aromatique adjacent un cycle à 5 ou 6 chainons, alors R5 ne représente pas un radical hydroxyle, quand R5 est en position ortho de la position $\alpha$ du cycle Ar de formule (a);
  - si R10 représente un atome d'hydrogène et R11 un radical hydroxyle, ou R10 et R11 forment un radical oxo unique de formule =O, R2 et R3 ne forment pas avec le cycle aromatique adjacent un cycle à 5 ou 6 chainons et Ar représente un radical de formula (a), (b), (c) ou (e), alors R1 ne représente pas (i), (ii) ou (iv), quand R6 représente un radical alkyle en C1-C6;

ainsi que leurs sels, et leurs isomères optiques et géométriques.

**2.** Composés selon la revendication 1, caractérisés en ce qu'ils se présentent sous forme de sels d'un métal alcalin ou alcalino-terreux, ou encore de zinc ou d'une amine organique.

**3.** Composés selon l'une des revendications 1 ou 2, caractérisés en ce que les radicaux alkyles en C1-C6 sont choisis dans le groupe constitué par les radicaux méthyle, éthyle, isopropyle, butyle, tertiobutyle et hexyle.

**4.** Composés selon l'une quelconque des revendications précédentes caractérisés en ce que les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyl.

**5.** Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

**6.** Composés selon l'une quelconque des revendications précédentes, caractérisés en ce que les radicaux polyhydroxyalkyles sont choisis dans le groupe constitué par les radicaux 2,3-dihydroxypropyle$_1$ 2,3,4-trihydroxybutyle, 2,3,4,5-tétrahydroxypentyle ou le reste du pentaérythritol.

**7.** Composés selon l'une quelconque des revendications précédentes, caractérisés en ce que le radical aryle est un radical phényle éventuellement substitué par au moins un atome d'halogène, un hydroxyle ou une fonction nitro.

**8.** Composés selon l'une quelconque des revendications précédentes, caractérisés en ce que les radicaux aralkyles sont choisis dans le groupe constitué par les radicauxl benzyle ou phénéthyle éventuellement substitués par au moins un atome d'halogène, un hydroxyle ou une fonction nitro.

**9.** Composés selon l'une quelconque des revendications précédentes, caractérisés en ce que les radicaux alkényles sont choisis dans le groupe constitué par les radicaux contenant jusqu'à 5 atomes de carbone et présentant une ou plusieurs insaturations éthyléniques, en particulier le radical allyle.

**10.** Composés selon l'une quelconque des revendications précédentes, caractérisés en ce que les restes de sucre sont choisis dans le groupe constitué par les restes de glucose, de galactose, de mannose ou d'acide glucuronique.

**11.** Composés selon l'une quelconque des revendications précédentes, caractérisés en ce que les restes d'aminoacide sont choisis dans le groupe constitué par les restes dérivant de la lysine, de la glycine ou de l'acide aspartique.

**12.** Composés selon l'une quelconque des revendications précédentes caractérisés en ce que les restes de peptides sont choisis dans le groupe constitué par les restes de dipeptides ou de tripeptides.

**13.** Composés selon l'une quelconque des revendications précédentes, caractérisés en ce que les radicaux hétérocycliques sont choisis dans le groupe constitué par les radicaux pipéridino, morpholino, pyrrolidino ou pipérazino,

éventuellement substitués en position 4 par un radical alkyle en $C_1$-$C_6$ ou mono ou polyhydroxyalkyle.

**14.** Composés selon l'une quelconque des revendications précédentes caractérisés en ce que les atomes d'halogènes sont choisis dans le groupe constitué par le fluor, le chlore et le brome.

**15.** Composés selon la revendication 1, caractérisés par le fait qu'ils sont pris dans le groupe constitué par :

- 4-[3-oxo-3-(5,6,7,8-tétrahydro-5,5,8,8-tétraméthyl-2-naphtyl)-1-propynyl]benzoate de méthyle
- 4-[3-hydroxy-3-(5,6,7,8-tétrahydro-5,5,8,8-tétraméthyl-2-naphtyl)-1-propynyl]benzoate de méthyle
- Acide 4-[3-hydroxy-3-(5,6,7,8-tétrahydro-5,5,8,8-tétraméthyl-2-naphtyl)-1-propynyl[benzoïque
- Acide 4-[3-oxo-3-(5,6,7,8-tétrahydro-5,5,8,8-tétraméthyl-2-naphtyl)-1-propynyl]benzoïque
- Acide 2-hydroxy-4-[3-oxo-3-(5,6,7,8-tétrahydro-5,5,8,8-tétraméthyl-2-naphtyl)-1-propynyl]benzoïque
- 2-hydroxy-4-[3-hydroxy-3-(5,6,7,8-tétrahydro-5,5,8,8-tétraméthyl-2-naphtyl)-1-propynyl]benzoate de méthyle
- Acide 2-hydroxy-4-[3-hydroxy-3-(5,6,7,8-tétrahydro-5,5,8,8-tétraméthyl-2-naphtyl)-1-propynyl]benzoïque
- Acide 2-hydroxy-4-[3-oxo-3-(3-tert-butyl-4-méthoxyphényl)-1-propynyl]benzoïque
- Acide 4-[1-oxo-3-(5,6,7,8-tétrahydro-5,5,8,8-tétraméthyl-2-naphtyl)-2-propynyl]benzoïque
- Acide 4-[1-hydroxy-3-(5,6,7,8-tétrahydro-5,5,8,8-tétraméthyl-2-naphtyl)-2-propynyl]benzoïque
- 2-hydroxy-4-[3-hydroxy-3-(4,4-diméthylthiochroman-6-yl)-1-propynyl]benzoate de méthyle
- Acide 2-hydroxy-4-[3-oxo-3-(4,4-diméthylthiochroman-6-yl)-1-propynyl]benzoïque
- Acide 4-[3-hydroxy-3-(3-tert-butyl-4-méthoxyphényl)-1-propynyl]benzoïque
- N-méthyl-4-[3-hydroxy-3-(5,6,7,8-tétrahydro-5,5,8,8-tétraméthyl-2-naphtyl)-1-propynyl]-2-pyrrole carboxylate de méthyle
- Acide N-méthyl-4-[3-hydroxy-3-(5,6,7,8-tétrahydro-5,5,8,8-tétraméthyl-2-naphtyl)-1-propynyl]-2-pyrrole carboxylique
- 4-[3-hydroxy-3-(5,6,7,8-tétrahydro-5,5,8,8-tétraméthyl-2-naphtyl)-1-propynyl]-2-pyrrole carboxylate de méthyle
- 2-hydroxy-4-[3-hydroxy-3-(4,4-diméthylthiochroman-7-yl)-1-propynyl]benzoate de méthyle
- Acide 4-[1-hydroxy-3-(4,4-diméthylthiochroman-6-yl)-2-propynyl]benzoïque
- Acide 2-hydroxy-4-[3-hydroxy-3-(4,4-diméthylthiochroman-6-yl)-1-propynyl]benzoïque
- Acide 2-hydroxy-4-[3-hydroxy-3-(4,4-diméthylthiochroman-7-yl)-1-propynyl]benzoïque
- Isomère (+) du 4-[3-hydroxy-3-(5,6,7,8-tétrahydro-5,5,8,8-tétraméthyl-2-naphtyl)-1-propynyl]benzoate de méthyle
- Isomère (-) de l'acide 4-[3-hydroxy-3-(5,6,7,8-tétrahydro-5,5,8,8-tétraméthyl-2-naphtyl)-1-propynyl]benzoïque
- Isomère (+) du 2-hydroxy-4-[3-hydroxy-3-(5,6,7,8-tétrahydro-5,5,8,8-tétraméthyl-2-naphtyl)-1-propynyl]benzoate de méthyle
- Isomère (-) de l'acide 2-hydroxy-4-[3-hydroxy-3-(5,6,7,8-tétrahydro-5,5,8,8-tétraméthyl-2-naphtyl)-1-propynyl]benzoïque

- Isomère (-) du 4-[3-hydroxy-3-(5,6,7,8-tétrahydro-5,5,8,8-tétraméthyl-2-naphtyl)-1-propynyl]benzoate de méthyle
- Isomère (-) du 2-hydroxy-4-[3-hydroxy-3-(5,6,7,8-tétrahydro-5,5,8,8-tétraméthyl-2-naphtyl)-1-propynyl]benzoate de méthyle
- Isomère (+) de l'acide 4-[3-hydroxy-3-(5,6,7,8-tétrahydro-5,5,8,8-tétraméthyl-2-naphtyl)-1-propynyl]benzoïque
- Isomère (+) de l'acide 2-hydroxy-4-[3-hydroxy-3-(5,6,7,8-tétrahydro-5,5,8,8-tétraméthyl-2-naphtyl)-1-propynyl]benzoïque
- 2-hydroxy-4-[3-hydroxy-3-méthyl-3-(5,6,7,8-tétrahydro-5,5,8,8-tétraméthyl-2-naphtyl)-1-propynyl]benzoate de méthyle
- Acide 2-hydroxy-4-[3-hydroxy-3-méthyl-3-(5,6,7,8-tétrahydro-5,5,8,8-tétraméthyl-2-naphtyl)-1-propynyl]benzoïque
- Acide 2-méthoxy-4-[3-hydroxy-3-(5,6,7,8-tétrahydro-5,5,8,8-tétraméthyl-2-naphtyl)-1-propynyl]benzoïque
- 4-[3-hydroxy-3-(5,6,7,8-tétrahydro-5,5,8,8-tétraméthyl-2-naphtyl)-1-propynyl]benzaldéhyde
- Acétate de 4-[3-hydroxy-3-(5,6,7,8-tétrahydro-5,5,8,8-tétraméthyl-2-naphtyl)-1-propynyl]benzyle
- 4-[3-hydroxy-3-(5,6,7,8-tétrahydro-5,5,8,8-tétraméthyl-2-naphtyl)-1-propynyl]benzeneméthanol
- 4-[3-hydroxy-3-(5,6,7,8-tétrahydro-5,5,8,8-tétraméthyl-2-naphtyl)-1-propynyl]toluène
- Acetate de 4-[3-hydroxy-3-(5,6,7,8-tétrahydro-3,5,5,8,8-pentaméthyl-2-naphtyl)-1-propynyl]phényle
- 4-[3-hydroxy-3-(5,6,7,8-tétrahydro-3,5,5,8,8-pentaméthyl-2-naphtyl)-1-propynyl]phénol
- 4-[3-hydroxy-3-(5,6,7,8-tétrahydro-5,5,8,8-tétraméthyl-2-naphtyl)-1-propynyl]phénylsulfinylméthane
- 4-[3-hydroxy-3-(5,6,7,8-tétrahydro-5,5,8,8-tétraméthyl-2-naphtyl)-1-propynyl]phénylsulfonylméthane
- N-éthyl-4-[3-hydroxy-3-(5,6,7,8-tétrahydro-5,5,8,8-tétraméthyl-2-naphtyl)-1-propynyl]benzamide
- N,N'-diéthyl-4-[3-hydroxy-3-(5,6,7,8-tétrahydro-5,5,8,8-tétraméthyl-2-naphtyl)-1-propynyl]benzamide
- Morpholide de l'acide 4-[3-hydroxy-3-(5,6,7,8-tétrahydro-5,5,8,8-tétraméthyl-2-naphtyl)-1-propynyl]benzoïque
- 5-[3-hydroxy-3-(5,6,7,8-tétrahydro-5,5,8,8-tétraméthyl-2-naphtyl)-1-propynyl]2-thiophènecarboxylate de méthyle
- 2-hydroxy-4-[3-hydroxy-3-(5,6,7,8-tétrahydro-3,5,5,8,8-pentaméthyl-2-naphtyl)-1-propynyl]benzoate de méthyle
- Acide 2-hydroxy-4-[3-hydroxy-3-(5,6,7,8-tétrahydro-3,5,5,8,8-pentaméthyl-2-naphtyl)-1-propynyl]benzoïque
- 2-hydroxy-4-[3-hydroxy-3-(3-méthoxy-5,6,7,8-tétrahydro-5,5,8,8-tétraméthyl-2-naphtyl)-1-propynyl]benzoate de méthyle
- Acide 2-hydroxy-4-[3-hydroxy-3-(3-méthoxy-5,6,7,8-tétrahydro-5,5,8,8-tétraméthyl-2-naphtyl)-1-propynyl]benzoïque
- Acide 4-[3-hydroxy-3-(4,4-diméthylthiochroman-6-yl)-1-propynyl]benzoïque

- Acide 4-[3-hydroxy-3-(4,4-diméthylthiochroman-7-yl)-1-propynyl]benzoïque
- 3-méthyl-4-[3-hydroxy-3-(5,6,7,8-tétrahydro-5,5,8,8-tétraméthyl-2-naphtyl)-1-propynyl]benzoate de méthyle
- Acide 3-méthyl-4-[3-hydroxy-3-(5,6,7,8-tétrahyro-5,5,8,8-tétraméthyl-2-naphtyl)-1-propynyl]benzoïque
- Acide 2-chloro-4-[3-hydroxy-3-(5,6,7,8-tétrahydro-5,5,8,8-tétraméthyl-2-naphtyl)-1-propynyl]benzoïque
- Acide 2-acétoxy-4-[3-acétoxy-3-(5,6,7,8-tétrahydro-5,5,8,8-tétraméthyl-2-naphtyl)-1-propynyl]benzoïque
- 4-[3-hydroxy-3-(3-tert-butyl-4-propyloxyphényl)-1-propynyl]benzoate de méthyle
- 4[3-hydroxy-3-(3-tert-butyl-4-héxyloxyphényl)-1-propynyl]benzoate de méthyle

**16.** Composés selon la revendication 1, caractérisés en ce que $R_5$ représente le groupement -OH, $R_7$ représente un radical $OR_8$, et $R_{11}$ représente un radical $-OR_6$, $R_6$ et $R_8$ ayant la signification donnée ci-dessus.

**17.** Composés selon l'une quelconque des revendications précédentes pour une utilisation comme médicament.

**18.** Composés selon la revendication 17 pour une utilisation comme médicament destiné au traitement des affections dermatologiques, rhumatismales, respiratoires, cardiovasculaires et ophtalmologiques.

**19.** Utilisation de l'un au moins des composés définis aux revendications 1 à 16 pour la fabrication d'un médicament destiné au traitement des affections dermatologiques, rhumatismales, respiratoires, cardiovasculaires et ophtalmologiques.

**20.** Composition pharmaceutique, caractérisée par le fait qu'elle comprend, dans un support pharmaceutiquement acceptable, au moins un des composés tels que définis à l'une quelconque des revendications 1 à 16.

**21.** Composition selon la revendication 20, caractérisée en ce que la concentration en composé(s) selon l'une des revendication 1 à 16 est comprise entre 0,001 % et 5 % en poids par rapport à l'ensemble de la composition.

**22.** Composition cosmétique, caractérisée par le fait qu'elle comprend, dans un support cosmétiquement acceptable, au moins un des composés tels que définis à l'une quelconque des revendications 1 à 16.

**23.** Composition selon la revendication 22, caractérisée en ce que la concentration en composé(s) selon l'une des revendications 1 à 16 est comprise entre 0,001 % et 3 % en poids par rapport à l'ensemble de la composition.

**24.** Utilisation d'une composition cosmétique telle que définie à l'une des revendications 22 ou 23 pour l'hygiène corporelle ou capillaire.

## Claims

**1.** Biaromatic propynyl compounds, characterized in that they correspond to the following general formula (I):

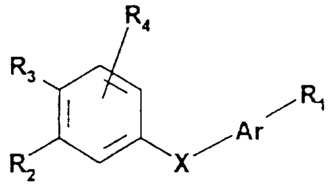

(I)

in which:

- * Ar represents a radical chosen from the radicals of following formulae (a)-(e)

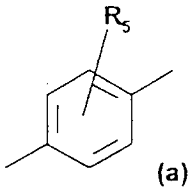
(a)
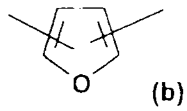
(b)
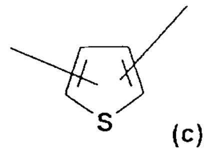
(c)

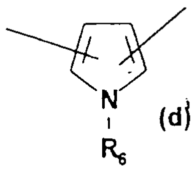
(d)
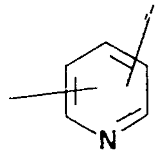
(e)

$R_5$ and $R_6$ having the meaning given below,

* $R_1$ represents:

(i) a hydrogen atom
(ii) a radical $-CH_3$
(iii) a radical $-CH_2-O-R_6$
(iv) a radical $-O-R_6$
(v) a radical $-CO-R_7$,
(vi) a radical $-S(O)_tR_9$

$R_6$, $R_7$, $R_9$ and t having the meaning given below,

* X represents a radical of formula:

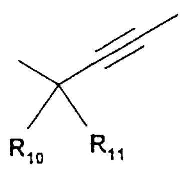
or
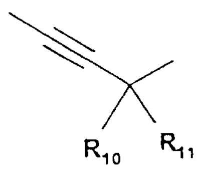

$R_{10}$ and $R_{11}$ having the meaning given below,

* $R_2$ and $R_3$ represent a hydrogen atom, a linear or branched alkyl radical having 1 to 20 carbon atoms, a radical $-OR_6$ or a radical $-SR_6$, $R_6$ having the meaning given below, it being understood that $R_2$ and $R_3$, taken together, can form with the adjacent aromatic ring a 5- or 6-membered ring optionally substituted by methyl groups and/or optionally interrupted by an oxygen or sulphur atom,

* $R_4$ represents a hydrogen atom, a halogen atom, a $C_1$-$C_6$ alkyl radical or a radical $-OR_6$, $R_6$ having the meaning given below,

it being understood that in all that precedes:

- $R_5$ has the same meaning as $R_4$,
- $R_6$ represents a hydrogen atom, a $C_1$-$C_6$ alkyl radical or a radical $-CO-R_9$, $R_9$ having the meaning given below,
- $R_7$ represents:

(a) a hydrogen atom
(b) a $C_1$-$C_6$ alkyl radical
(c) a radical of formula:

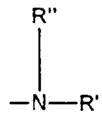

R' and R" having the meaning given below,

(d) a radical $-OR_8$, $R_8$ having the meaning given below,

- $R_8$ represents a hydrogen atom, a linear or branched alkyl radical having 1 to 20 carbon atoms, an alkenyl radical, a mono- or polyhydroxyalkyl radical, an optionally substituted aryl or aralkyl radical or a sugar residue or an amino acid or peptide residue,
- $R_9$ represents a $C_1$-$C_6$ alkyl radical,
- $R_{10}$ represents a hydrogen atom, a $C_1$-$C_6$ alkyl radical or a radical $-OR_6$,
- $R_{11}$ represents a radical $-OR_6$,
- R' and R" represent a hydrogen atom, a $C_1$-$C_6$ alkyl radical, a mono- or polyhydroxyalkyl radical, an optionally substituted aryl radical or an amino acid or peptide or sugar residue or alternatively, taken together, form a heterocycle,
- t is an integer equal to 0, 1 or 2,
- the radicals $R_{10}$ and $R_{11}$ above, taken together, can form a single oxo radical of formula =O, with the following conditions:

  - if $R_{10}$ represents a hydrogen atom, $R_{11}$ a hydroxyl radical and $R_2$ and $R_3$ do not form, with the adjacent aromatic ring, a 5- or 6-membered ring, then $R_4$ does not represent a hydroxyl radical when $R_4$ is in a position ortho to the $\alpha$ position of the ring;
  - if $R_{10}$ represents a hydrogen atom and $R_{11}$ a hydroxyl radical, or $R_{10}$ and $R_{11}$ form a single oxo radical of formula =O, and $R_2$ and $R_3$ do not form, with the adjacent aromatic ring, a 5- or 6-membered ring, then $R_5$ does not represent a hydroxyl radical when $R_5$ is in a position ortho to the $\alpha$ position of the Ar ring of formula (a);
  - if $R_{10}$ represents a hydrogen atom and $R_{11}$ a hydroxyl radical, or $R_{10}$ and $R_{11}$ form a single oxo radical of formula =O, $R_2$ and $R_3$ do not form, with the adjacent aromatic ring, a 5- or 6-membered ring and Ar represents a radical of formula (a), (b), (c) or (e), then $R_1$ does not represent (i), (ii) or (iv) when $R_6$ represents a $C_1$-$C_6$ alkyl radical;

as well as their salts and their optical and geometric isomers.

**2.** Compounds according to Claim 1, characterized in that they are in the form of salts of an alkali or alkaline-earth metal, or alternatively of zinc or of an organic amine.

**3.** Compounds according to one of Claims 1 or 2, characterized in that the $C_1$-$C_6$ alkyl radicals are chosen from the group consisting of methyl, ethyl, isopropyl, butyl, tert-butyl and hexyl radicals.

**4.** Compounds according to any one of the preceding claims, characterized in that the linear or branched alkyl radicals having 1 to 20 carbon atoms are chosen from the group consisting of methyl, ethyl, propyl, 2-ethylhexyl, octyl, dodecyl, hexadecyl and octadecyl radicals.

**5.** Compounds according to any one of the preceding claims, characterized in that the monohydroxyalkyl radicals are chosen from the group consisting of 2-hydroxyethyl, 2-hydroxypropyl or 3-hydroxypropyl radicals.

**6.** Compounds according to any one of the preceding claims, characterized in that the polyhydroxyalkyl radicals are chosen from the group consisting of 2,3-dihydroxypropyl, 2,3,4-trihydroxybutyl or 2,3,4,5-tetrahydroxypentyl radicals or the pentaerythritol residue.

**7.** Compounds according to any one of the preceding claims, characterized in that the aryl radical is a phenyl radical optionally substituted by at least one halogen atom, a hydroxyl or a nitro functional group.

**8.** Compounds according to any one of the preceding claims, characterized in that the aralkyl radicals are chosen

from the group consisting of benzyl or phenethyl radicals optionally substituted by at least one halogen atom, a hydroxyl or a nitro functional group.

**9.** Compounds according to any one of the preceding claims, characterized in that the alkenyl radicals are chosen from the group consisting of the radicals containing up to 5 carbon atoms and having one or more ethylenic unsaturations, in particular the allyl radical.

**10.** Compounds according to any one of the preceding claims, characterized in that the sugar residues are chosen from the group consisting of glucose, galactose, mannose or glucuronic acid residues.

**11.** Compounds according to any one of the preceding claims, characterized in that the amino acid residues are chosen from the group consisting of residues derived from lysine, glycine or aspartic acid.

**12.** Compounds according to any one of the preceding claims, characterized in that the peptide residues are chosen from the group consisting of dipeptide or tripeptide residues.

**13.** Compounds according to any one of the preceding claims, characterized in that the heterocyclic radicals are chosen from the group consisting of piperidino, morpholino, pyrrolidino or piperazino radicals which are optionally substituted at position 4 by a $C_1$-$C_6$ alkyl radical or a mono- or polyhydroxyalkyl radical.

**14.** Compounds according to any one of the preceding claims, characterized in that the halogen atoms are chosen from the group consisting of fluorine, chlorine and bromine.

**15.** Compounds according to Claim 1, characterized in that they are taken from the group consisting of:

- Methyl 4-[3-oxo-(5,6,7,8-tetrahydro-5,5,8,8-tetramethyl-2-naphthyl)-1-propynyl]benzoate
- Methyl 4-[3-hydroxy-3-(5,6,7,8-tetrahydro-5,5,8,8-tetramethyl-2-naphthyl)-1-propynyl]benzoate
- 4-[3-Hydroxy-3-(5,6,7,8-tetrahydro-5,5,8,8-tetramethyl-2-naphthyl)-1-propynyl]benzoic acid
- 4-[3-Oxo-3-(5,6,7,8-tetrahydro-5,5,8,8-tetramethyl-2-naphthyl)-1-propynyl]benzoic acid
- 2-Hydroxy-4-[3-oxo-3-(5,6,7,8-tetrahydro-5,5,8,8-tetramethyl-2-naphthyl)-1-propynyl]benzoic acid
- Methyl 2-hydroxy-4-[3-hydroxy-3-(5,6,7,8-tetrahydro-5,5,8,8-tetramethyl-2-naphthyl)-1-propynyl]benzoate
- 2-Hydroxy-4-[3-hydroxy-3-(5,6,7,8-terranydro-5,5,8,8-tetramethyl-2-naphthyl)-1-propynyl]benzoic acid
- 2-Hydroxy-4-[3-oxo-3-(3-tert-butyl-4-methoxyphenyl)-1-propynyl]benzoic acid
- 4-[1-Oxo-3-(5,6,7,8-tetrahydro-5,5,8,8-tetramethyl-2-naphthyl)-2-propynyl]benzoic acid
- 4-[1-Hydroxy-3-(5,6,7,8-tetrahydro-5,5,8,8-tetramethyl-2-naphthyl)-2-propynyl]benzoic acid
- Methyl 2-hydroxy-4-[3-hydroxy-3-(4,4-dimethylthiochroman-6-yl)-1-propynyl]benzoate
- 2-Hydroxy-4-[3-oxo-3-(3,4-dimethylthiochroman-6-yl)-1-propynyl]benzoic acid
- 4-[3-Hydroxy-3-(3-tert-butyl-4-methoxyphenyl)-1-propynyl]benzoic acid
- Methyl N-methyl-4-[3-hydroxy-3-(5,6,7,8-tetrahydro-5,5,8,8-tetramethyl-2-naphthyl)-1-propynyl]-2-pyrrolecarboxylate
- N-Methyl-4-[3-hydroxy-3-(5,6,7,8-tetrahydro-5,5,8,8-tetramethyl-2-naphthyl)-1-propynyl]-2-pyrrolecarboxylic acid
- Methyl 4-[3-hydroxy-3-(5,6,7,8-tetrahydro-5,5,8,8-tetramethyl-2-naphthyl)-1-propynyl]-2-pyrrolecarboxylate
- Methyl 2-hydroxy-4-[3-hydroxy-3-(4,4-dimethylthiochroman-7-yl)-1-propynyl]benzoate
- 4-[1-Hydroxy-3-(4,4-dimethylthiochroman-6-yl)-2-propynyl]benzoic acid
- 2-Hydroxy-4-[3-hydroxy-3-(4,4-dimethylthiochroman-6-yl)-1-propynyl]benzoic acid
- 2-Hydroxy-4-[3-hydroxy-3-(4,4-dimethylthiochroman-7-yl)-1-propynyl]benzoic acid
- (+)-Isomer of methyl 4-[3-hydroxy-3-(5,6,7,8-tetrahydro-5,5,8,8-tetramethyl-2-naphthyl)-1-propynyl]benzoate,
- (-)-Isomer of 4-[3-hydroxy-3-(5,6,7,8-tetrahydro-5,5,8,8-tetramethyl-2-naphthyl)-1-propynyl]benzoic acid
- (+)-Isomer of methyl-2-hydroxy-4-[3-hydroxy-3-(5,6,7,8-tetrahydro-5,5,8,8-tetramethyl-2-naphthyl)-1-propynyl]benzoate,
- (-)-Isomer of 2-hydroxy-4-[3-hydroxy-3-(5,6,7,8-tetrahydro-5,5,8,8-tetramethyl-2-naphthyl)-1-propynyl]benzoic acid
- (-)-Isomer of methyl 4-[3-hydroxy-3-(5,6,7,8-tetrahydro-5,5,8,8-tetramethyl-2-naphthyl)-1-propynyl]benzoate
- (-)-Isomer of methyl 2-hydroxy-4-[3-hydroxy-3-(5,6,7,8-tetrahydro-5,5,8,8-tetramethyl-2-naphthyl)-1-propynyl]benzoate
- (+)-Isomer of 4-[3-hydroxy-3-(5,6,7,8-tetrahydro-5,5,8,8-tetramethyl-2-naphthyl)-1-propynyl]benzoic acid
- (+)-Isomer of 2-hydroxy-4-[3-hydroxy-3-(5,6,7,8-tetrahydro-5,5,8,8-tetramethyl-2-naphthyl)-1-propynyl]benzoic

acid

- Methyl 2-hydroxy-4-[3-hydroxy-3-methyl-3-(5,6,7,8-tetrahydro-5,5,8,8-tetramethyl-2-naphthyl)-1-propynyl]benzoate
- 2-Hydroxy-4-[3-hydroxy-3-methyl-3-(5,6,7,8-tetrahydro-5,5,8,8-tetramethyl-2-naphthyl)-1-propynyl]benzoic acid
- 2-Methoxy-4-[3-hydroxy-3-(5,6,7,8-tetrahydro-5,5,8,8-tetramethyl-2-naphthyl)-1-propynyl]benzoic acid
- 4-[3-Hydroxy-3-(5,6,7,8-tetrahydro-5,5,8,8-tetramethyl-2-naphthyl)-1-propynyl]benzaldehyde
- 4-[3-Hydroxy-3-(5,6,7,8-tetrahydro-5,5,8,8-tetramethyl-2-naphthyl)-1-propynyl]benzyl acetate
- 4-[3-Hydroxy-3-(5,6,7,8-tetrahydro-5,5,8,8-tetramethyl-2-naphthyl)-1-propynyl]benzene methanol
- 4-[3-Hydroxy-3-(5,6,7,8-tetrahydro-5,5,8,8-tetramethyl-2-naphthyl)-1-propynyl]toluene
- 4-[3-Hydroxy-3-(5,6,7,8-tetrahydro-3,5,5,8,8-pentamethyl-2-naphthyl)-1-propynyl]phenyl acetate
- 4-[3-Hydroxy-3-(5,6,7,8-tetrahydro-3,5,5,8,8-pentamethyl-2-naphthyl)-1-propynyl]phenol
- 4-[3-Hydroxy-3-(5,6,7,8-tetrahydro-5,5,8,8-tetramethyl-2-naphthyl)-1-propynyl]phenylsulphinylmethane
- 4-[3-Hydroxy-3-(5,6,7,8-tetrahydro-5,5,8,8-tetramethyl-2-naphthyl)-1-propynyl]phenylsulphonylmethane
- N-Ethyl-4-[3-hydroxy-3-(5,6,7,8-tetrahydro-5,5,8,8-tetramethyl-2-naphthyl)-1-propynyl]benzamide
- N,N'-Diethyl-4-[3-hydroxy-3-(5,6,7,8-tetrahydro-5,5,8,8-tetramethyl-2-naphthyl)-1-propynyl]benzamide
- Morpholide of 4-[3-hydroxy-3-(5,6,7,8-tetrahydro-5,5,8,8-tetramethyl-2-naphthyl)-1-propynyl]benzoic acid
- Methyl 5-[3-hydroxy-3-(5,6,7,8-tetrahydro-5,5,8,8-tetramethyl-2-naphthyl)-1-propynyl]-2-thiophenecarboxylate
- Methyl 2-hydroxy-4-[3-hydroxy-3-(5,6,7,8-tetrahydro-3,5,5,8,8-pentamethyl-2-naphthyl)-1-propynyl]benzoate
- 2-Hydroxy-4-[3-hydroxy-3-(5,6,7,8-tetrahydro-3,5,5,8,8-pentamethyl-2-naphthyl)-1-propynyl]benzoic acid
- Methyl 2-hydroxy-4-[3-hydroxy-3-(3-methoxy-5,6,7,8-tetrahydro-5,5,8,8-tetramethyl-2-naphthyl)-1-propynyl]benzoate
- 2-Hydroxy-4-[3-hydroxy-3-(3-methoxy-5,6,7,8-tetrahydro-5,5,8,8-tetramethyl-2-naphthyl)-1-propynyl]benzoic acid
- 4-[3-Hydroxy-3-(4,4-dimethylthiochroman-6-yl)-1-propynyl]benzoic acid
- 4-[3-Hydroxy-3-(4,4-dimethylthiochroman-7-yl)-1-propynyl]benzoic acid
- Methyl 3-methyl-4-[3-hydroxy-3-(5,6,7,8-tetrahydro-5,5,8,8-tetramethyl-2-naphthyl)-1-propynyl]benzoate
- 3-Methyl-4-[3-hydroxy-3-(5,6,7,8-tetrahydro-5,5,8,8-tetramethyl-2-naphthyl)-1-propynyl]benzoic acid
- 2-Chloro-4-[3-hydroxy-3-(5,6,7,8-tetrahydro-5,5,8,8-tetramethyl-2-naphthyl)-1-propynyl]benzoic acid
- 2-Acetoxy-4-[3-acetoxy-3-(5,6,7,8-tetrahydro-5,5,8,8-tetramethyl-2-naphthyl)-1-propynyl]benzoic acid
- Methyl 4-[3-hydroxy-3-(3-tert-butyl-4-propyloxyphenyl)-1-propynyl]benzoate
- Methyl 4-[3-hydroxy-3-(3-tert-butyl-4-hexyloxyphenyl)-1-propynyl]benzoate.

**16.** Compounds according to Claim 1, characterized in that $R_5$ represents the group -OH, $R_7$ represents a radical $OR_8$ and $R_{11}$ represents a radical $-OR_6$, $R_6$ and $R_8$ having the meaning given above.

**17.** Compounds according to any one of the preceding claims, for use as medicinal products.

**18.** Compounds according to Claim 17, for use as medicinal products intended for the treatment of dermatological, rheumatic, respiratory, cardiovascular and ophthalmological conditions.

**19.** Use of at least one of the compounds defined in Claims 1 to 16 for the manufacture of a medicinal product intended for the treatment of dermatological, rheumatic, respiratory, cardiovascular and ophthalmological conditions.

**20.** Pharmaceutical composition, characterized by the fact that it comprises, in a pharmaceutically acceptable carrier, at least one of the compounds as defined in any one of Claims 1 to 16.

**21.** Composition according to Claim 20, characterized in that the concentration of compound(s) according to one of Claims 1 to 16 is between 0.001% and 5% by weight relative to the whole composition.

**22.** Cosmetic composition, characterized by the fact that it comprises, in a cosmetically acceptable carrier, at least one of the compounds as defined in any one of Claims 1 to 16.

**23.** Composition according to Claim 22, characterized in that the concentration of compound(s) according to one of Claims 1 to 16 is between 0.001% and 3% by weight relative to the whole composition.

**24.** Use of a cosmetic composition as defined in one of Claims 22 or 23 for body or hair care.

## Patentansprüche

1. Biaromatische Propinylverbindungen,
**dadurch gekennzeichnet, daß**
sie der folgenden allgemeinen Formel (I) entsprechen:

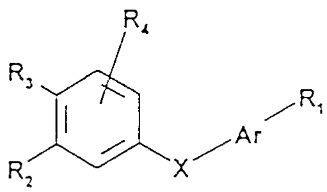

(I),

worin bedeuten:

- Ar eine Gruppe, die unter den Gruppen der folgenden Formeln (a) bis (e) ausgewählt ist:

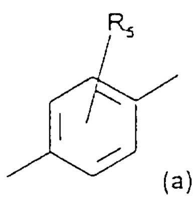
(a)

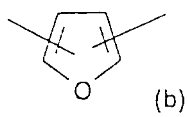
(b)

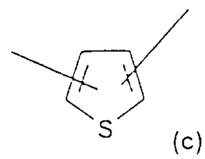
(c)

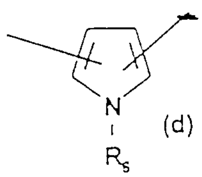
(d)

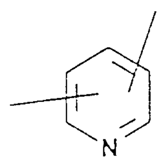
(e)

wobei $R_5$ und $R_6$ die nachfolgend angegebene Bedeutung aufweisen,

- $R_1$

(i) Wasserstoff,
(ii) die Gruppe $-CH_3$,
(iii) eine Gruppe $-CH_2-O-R_6$,
(iv) eine Gruppe $-O-R_6$,
(v) eine Gruppe $-CO-R_7$,
(vi) eine Gruppe $-S(O)_t-R_9$,

wobei $R_6$ $R_7$, $R_9$ und t die nachstehend angegebenen Bedeutungen aufweisen,

- X eine Gruppe der Formel

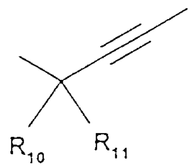

oder

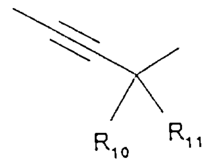

,

wobei $R_{10}$ und $R_{11}$ die nachstehend angegebenen Bedeutungen aufweisen,

- $R_2$ und $R_3$ ein Wasserstoffatom, eine geradkettige oder verzweigte Alkylgruppe mit 1 bis 20 Kohlenstoffatomen, eine Gruppe $-OR_6$ oder eine Gruppe $-SR_6$, wobei $R_6$ die nachfolgend angegebene Bedeutung aufweist, mit der Maßgabe, daß $R_2$ und $R_3$ gemeinsam mit dem angrenzenden aromatischen Ring einen 5- oder 6-Ring bilden können, der gegebenenfalls mit Methylgruppen substituiert und/oder durch ein Sauerstoff- oder Schwefelatom unterbrochen sein kann.

- $R_4$ ein Wasserstoff- oder Halogenatom, eine $C_{1-6}$-Alkylgruppe oder eine Gruppe $-OR_6$, wobei $R_6$ die nachfolgend angegebene Bedeutung aufweist,
mit der Maßgabe, daß im folgenden bedeuten:

  - $R_5$ die Bedeutungen von $R_4$,
  - $R_6$ Wasserstoff, eine $C_{1-6}$-Alkylgruppe oder eine Gruppe $-CO-R_9$, wobei $R_9$ die nachfolgend angegebene Bedeutung aufweist,
  - $R_7$

    (a) ein Wasserstoffatom,
    (b) eine $C_{1-6}$-Alkylgruppe
    (c) oder eine Gruppe der Formel:

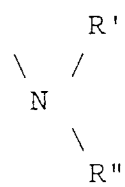

    worin R' und R" die nachfolgend angegebenen Bedeutungen aufweisen,
    (d) eine Gruppe $-OR_8$, wobei $R_8$ die nachfolgend angegebene Bedeutung aufweist,

  - $R_8$ Wasserstoff, eine geradkettige oder verzweigte Alkylgruppe mit 1 bis 20 Kohlenstoffatomen, eine Alkenylgruppe, eine Mono- oder Polyhydroxyalkylgruppe, eine Aryl- oder Aralkylgruppe, die gegebenenfalls substituiert sind, oder einen Zucker-, Aminosäure- oder Peptidrest,
  - $R_9$ eine $C_{1-6}$-Alkylgruppe,
  - $R_{10}$ Wasserstoff, eine $C_{1-6}$-Alkylgruppe oder eine Gruppe $-OR_6$,
  - $R_{11}$ eine Gruppe $-OR_6$,
  - R' und R" Wasserstoff, eine $C_{1-6}$-Alkylgruppe, eine Mono- oder Polyhydroxyalkylgruppe, eine Arylgruppe, die gegebenenfalls substituiert ist, oder einen Aminosäure-, Petid- oder Zuckerrest oder sie bilden gemeinsam einen Heterocyclus,
  - t Null,1 oder 2, und
  - die oben genannten Gruppen $R_{10}$ und $R_{11}$ können gemeinsam eine einzige Oxogruppe der Formel =O bilden,
  mit den folgenden Maßgaben, daß:
  - wenn $R_{10}$ Wasserstoff und $R_{11}$ Hydroxy bedeutet und $R_2$ und $R_3$ mit dem angrenzenden aromatischen

Ring keinen 5- oder 6-Ring bilden, $R_4$ nicht Hydroxy bedeuten kann, wenn sich $R_4$ in o-Stellung zur $\alpha$-Stellung des Rings befindet;

- wenn $R_{10}$ Wasserstoff und $R_{11}$ Hydroxy bedeutet oder $R_{10}$ und $R_{11}$ gemeinsam eine einzige Oxogruppe der Formel =O bilden und $R_2$ und $R_3$ mit dem angrenzenden aromatischen Ring keinen 5- oder 6-Ring bilden, $R_5$ keine Hydroxygruppe bedeuten kann, wenn sich $R_5$ in o-Stellung zur $\alpha$-Stellung des Rings Ar der Formel (a) befindet;
- wenn $R_{10}$ Wasserstoff und $R_{11}$ Hydroxy bedeutet oder $R_{10}$ und $R_{11}$ gemeinsam eine einzige Oxogruppe der Formel =O bilden und $R_2$ und $R_3$ mit dem angrenzenden aromatischen Ring keinen 5- oder 6-Ring bilden und Ar eine Gruppe (a), (b), (c) oder (e) bedeutet, $R_1$ nicht (i), (ii) oder (iv) bedeuten kann, wenn $R_6$ eine $C_{1-6}$-Alkylgruppe bedeutet.

sowie deren Salze und optischen und geometrischen Isomeren.

**2.** Verbindungen nach Anspruch 1, dadurch gekennzeichnet, daß sie in Form eines Alkali- oder Erdalkalimetallsalzes oder auch in Form eines Zinksalzes oder Salzes eines organischen Amins vorliegen.

**3.** Verbindungen nach einem der Ansprüche 1 oder 2, dadurch gekennzeichnet, daß die $C_{1-6}$-Alkylgruppen unter Methyl, Ethyl, Isopropyl, Butyl, *tert.*-Butyl und Hexyl ausgewählt sind.

**4.** Verbindungen nach einem der vorhergehenden Ansprüche, dadurch gekennzeichnet, daß die geradkettigen oder verzweigten Alkylgruppen mit 1 bis 20 Kohlenstoffatomen unter Methyl, Ethyl, Propyl, 2-Ethylhexyl, Octyl, Dodecyl, Hexadecyl und Octadecyl ausgewählt sind.

**5.** Verbindungen nach einem der vorhergehenden Ansprüche, dadurch gekennzeichnet, daß die Monohydroxyalkylgruppen unter 2-Hydroxyethyl, 2-Hydroxypropyl oder 3-Hydroxypropyl ausgewählt sind.

**6.** Verbindungen nach einem der vorhergehenden Ansprüche, dadurch gekennzeichnet, daß die Polyhydroxyalkylgruppen unter 2,3-Dihydroxypropyl, 2,3,4-Trihydroxybutyl, 2,3,4,5-Tetrahydroxypentyl oder der Pentaerythritgruppe ausgewählt sind.

**7.** Verbindungen nach einem der vorhergehenden Ansprüche, dadurch gekennzeichnet, daß die Arylgruppe eine Phenylgruppe ist, die gegebenenfalls mit mindestens einem Halogenatom, einer Hydroxygruppe oder einer Nitrogruppe substituiert ist.

**8.** Verbindungen nach einem der vorhergehenden Ansprüche, dadurch gekennzeichnet, daß die Aralkylgruppen unter den Benzyl- oder Phenetylgruppen ausgewählt sind, die gegebenebfalls mit mindestens einem Halogenatom, einer Hydroxygruppe oder einer Nitrogruppe substituiert sind.

**9.** Verbindungen nach einem der vorhergehenden Ansprüche, dadurch gekennzeichnet, daß die Alkenylgruppen unter den Gruppen mit bis zu 5 Kohlenstoffatomen ausgewählt sind, die eine oder mehrere ethylenisch ungesättigte Doppelbindungen aufweisen, insbesondere Allyl.

**10.** Verbindungen nach einem der vorhergehenden Ansprüche, dadurch gekennzeichnet, daß die Zuckerreste unter den Resten von Glucose, Galactose, Mannose oder Glucuronsäure ausgewählt sind.

**11.** Verbindungen nach einem der vorhergehenden Ansprüche, dadurch gekennzeichnet, daß die Aminosäurereste unter den Resten ausgewählt sind, die von Lysin, Glycin oder Asparaginsäure abgeleitet sind.

**12.** Verbindungen nach einem der vorhergehenden Ansprüche, dadurch gekennzeichnet, daß die Peptidreste unter den Dipeptid- oder Tripeptidresten ausgewählt sind.

**13.** Verbindungen nach einem der vorhergehenden Ansprüche, dadurch gekennzeichnet, daß die heterocyclischen Gruppen unter Piperidino, Morpholino, Pyrrolidino oder Piperazino ausgewählt sind, die gegebenenfalls in 4-Stellung mit einer $C_{1-6}$-Alkylgruppe oder Mono- oder Polyhydroxyalkylgruppe substituiert sind.

**14.** Verbindungen nach einem der vorhergehenden Ansprüche, dadurch gekennzeichnet, daß die Halogenatome unter Fluor, Chlor und Brom ausgewählt sind.

**15.** Verbindungen nach Anspruch 1, dadurch gekennzeichnet, daß sie ausgewählt sind unter:

4-[3-Oxo-3-(5,6,7,8-tetrahydro-5,5,8,8-tetramethyl-2-naphthyl)-1-propinyl]-benzoesäuremethylester

4-[3-Hydroxy-3-(5,6,7,8-tetrahydro-5,5,8,8-tetramethyl-2-naphthyl)-1-propinyl]-benzoesäuremethylester

4-[3-Hydroxy-3-(5,6,7,8-tetrahydro-5,5,8,8-tetramethyl-2-naphthyl)-1-propinyl]-benzoesäure

4-[3-Oxo-3-(5,6,7,8-tetrahydro-5,5,8,8-tecramethyl-2-naphthyl)-1-propinyl]-benzoesäure

2-Hydroxy-4-[3-oxo-3-(5,6,7,8-tetrahydro-5,5,8,8-tetramethyl-2-naphthyl)-1-propinyl]-benzoesäure

2-Hydroxy-4-[3-hydroxy-3-(5,6,7,8-tetrahydro-5,5,8,8-tetramethyl-2-naphthyl)-1-propinyl]-benzoesäuremethylester

2-Hydroxy-4-[3-hydroxy-3-(5,6,7,8-tetrahydro-5,5,8,8-tetramethyl-2-naphthyl)-1-propinyl]-benzoesäure

2-Hydroxy-4-[3-oxo-3-(3-*tert.*-butyl-4-methoxphenyl)-1-propinyl]-benzoesäure

4-[1-Oxo-3-(5,6,7,8-tetrahydro-5,5,8,8-tetramethyl-2-naphthyl)-2-propinyl]-benzoesäure

4-[1-Hydroxy-3-(5,6,7,8-tetrahydro-5,5,8,8-tetramethyl-2-naphthyl)-2-propinyl]-benzoesäure

2-Hydroxy-4-[3-Hydroxy-3-(4,4-dimethylthiochroman-6-yl)-1-propinyl]-benziesäuremethylester

2-Hydroxy-4-[3-oxo-3-(4,4-dimethylthiochroman-6-yl)-1-propinyl]-benzoesäure

4-[3-Hydroxy-3-[3-*tert.*-butyl-4-methoxyphenyl)-1-propinyl]-benzoesäure

N-Methyl-4-[3-hydroxy-3-(5,6,7,8-tetrahydro-5,5,8,8-tetramethyl-2-naphthyl)-1-propinyl]-2-pyrrolmethylcarboxylat

N-Methyl-4-[3-hydroxy-3-(5,6,7,8-tetrahydro-5,5,8,8-tetramethyl-2-naphthyl)-1-propinyl]-2-pyrrolcarbonsäure

4-[3-Hydroxy-3-(5,6,7,8-tetrahydro-5,5,8,8-tetramethyl-2-naphthyl)-1-propinyl]-2-pyrrolmethylcarboxylat

2-Hydroxy-4-[3-hydroxy-3-(4,4-dimethylthiochroman-7-yl)-1-propinyl]-benzoesäuremethylester

4-[1-Hydroxy-3-(4,4-dimethylthiochroman-6-yl)-2-propinyl]-benzoesäure

2-Hydroxy-4-[3-hydroxy-3-(4,4-dimethylthiochroman-6-yl)-1-propinyl]-benzoesäure

2-Hydroxy-4-[3-hydroxy-3-(4,4-dimethylthiochroman-7-yl)-1-propinyl]-benzoesäure

(+) Isomer von 4-[3-Hydroxy-3-(5,6,7,8-tetrahydro-5,5,8,8-tetramethyl-2-naphthyl)-1-propinyl]-benzoesäuremethylester

(-) Isomer von 4-[3-Hydroxy-3-(5,6,7,8-tetrahydro-5,5,8,8-tetramethyl-2-naphthyl)-1-propinyl]-benzoesäure

(+) Isomer von 2-Hydroxy-4-[3-hydroxy-3-(5,6,7,8-tetrahydro-5,5,8,8-tetramethyl-2-naphthyl)-1-propinyl]-benzoesäuremethylester

(-) Isomer von 2-Hydroxy-4-[3-hydroxy-3-(5,6,7,8-tetrahydro-5,5,8,8-tetramethyl-2-naphthyl)-1-propinyl]-benzoesäure

(-) Isomer von 4-[3-Hydroxy-3-(5,6,7,8-tetrahydro-5,5,8,8-tetramethyl-2-naphthyl)-1-propinyl]-benzoesäuremethylester

(-) Isomer von 2-Hydroxy-4-[3-hydroxy-3-(5,6,7,8-tetrahydro-5,5,8,8-tetramethyl-2-naphthyl)-1-propinyl]-benzoesäuremethylester

(+) Isomer von 4-[3-Hydroxy-3-(5,6,7,8-tetrahydro-5,5,8,8-tetramethyl-2-naphthyl)-1-propinyl]-benzoesäure

(+) Isomer von 2-Hydroxy-4-[3-hydroxy-3-(5,6,7,8-tetrahydro-5,5,8,8-tetramethyl-2-naphthyl)-1-propinyl]-benzoesäure

2-Hydroxy-4-[3-hydroxy-3-methyl-3-(5,6,7,8-tetrahydro-5,5,8,8-tetramethyl-2-naphthyl)-1-propinyl]-benzoesäuremethylester

2-Hydroxy-4-[3-hydroxy-3-methyl-3-(5,6,7,8-tetrahydro-5,5,8,8,-tetramethyl-2-naphthyl)-1-propinyl]-benzoesäure

2-Methoxy-4-[3-hydroxy-3-(5,6,7,8-tetrahydro-5,5,8,8,-tetramethyt-2-naphthyl)-1-propinyl]-benzoesäure

4-[3-Hydroxy-3-(5,6,7,8-tetrahydro-5,5,8,8,-tetramethyl-2-naphthyl)-1-propinyl]-benzaldehyd

4-[3-Hydroxy-3-(5,6,7,8-tetrahydro-5,5,8,8,-tetramethyl-2-naphthyl)-1-propinyl]-benzylacetat

4-[3-Hydroxy-3-(5,6,7,8-tetrahydro-5,5,8,8,-tetramethyl-2-naphthyl)-1-propinyl]-benzolmethanol

4-[3-Hydroxy-3-(5,6,7,8-tetrahydro-5,5,8,8,-tetramethyl-2-naphthyl)-1-propinyl]-toluol

4-[3-Hydroxy-3-(5,6,7,8-tetrahydro-3,5,5,8,8,-pentamethyl-2-naphthyl)-1-propinyl]-phenylacetat

4-[3-Hydroxy-3-(5,6,7,8-tetrahydro-3,5,5,8,8,-pentamethyl-2-naphthyt)-1-propinyl]-phenol

4-[3-Hydroxy-3-(5,6,7,8-tetrahydro-5,5,8,8,-tetramethyl-2-naphthyl)-1-propinyl]-phenylsulfinylmethan

4-[3-Hydroxy-3-(5,6,7,8-tetrahydro-5,5,8,8,-tetramethyl-2-naphthyl)-1-propinyl]-phenylsulfonylmethan

N-Ethyl-4-[3-hydroxy-3-(5,6,7,8-tetrahydro-5,5,8,8,-tetramethyl-2-naphthyl)-1-propinyl]-benzamid

N,N'-Diethyl-4-[3-hydroxy-3-(5,6,7,8-tetrahydro-5,5,8,8,-tetramethyl-2-naphthyl)-1-propinyl]-benzamid

4-[3-Hydroxy-3-(5,6,7,8-tetrahydro-5,5,8,8,-tetramethyl-2-naphthyl)-1-propinyl]-benzoesäuremorpholid

5-[3-Hydroxy-3-(5,6,7,8-tetrahydro-5,5,8,8,-tetramethyl-2-naphthyl)-1-propinyl]-2-thiophenmethylcarboxylat

2-Hydroxy-4-[3-hydroxy-3-(5,6,7,8-tetrahydro-3,5,5,8,8,-pentamethyl-2-naphthyl)-1-propinyl]-benzoesäuremethylester

2-Hydroxy-4-[3-hydroxy-3-(5,6,7,8-tetrahydro-3,5,5,8,8,-pentamethyl-2-naphthyl)-1-propinyl]-benzoesäure

2-Hydroxy-4-[3-hydroxy-3-(3-methoxy-5,6,7,8-tetrahydro-5,5,8,8-tetramethyl-2-naphthyl)-1-propinyl]-benzoesäuremethylester

2-Hydroxy-4-[3-hydroxy-3-(3-methoxy-5,6,7,8-tetrahydro-5,5,8,8-tetramethyl-2-naphthyl)-1-propinyl]-benzoesäure

4-[3-Hydroxy-3-(4,4-dimethylthiochroman-6-yl)-1-propinyl]-benzoesäure

4-[3-Hydroxy-3-(4,4-dimethylthiochroman-7-yl)-1-propinyl]-benzoesäure

3-Methyl-4-[3-hydroxy-3-(5,6,7,8-tetrahydro-5,5,8,8,-tetramethyl-2-naphthyl)-1-propinyl]-benzoesäuremethylester

3-Methyl-4-[3-hydroxy-3-(5,6,7,8-tetrahydro-5,5,8,8,-tetramethyl-2-naphthyl)-1-propinyl]-benzoesäure

2-Chlor-4-[3-hydroxy-3-(5,6,7,8-tetrahydro-5,5,8,8,-tetramethyl-2-naphthyl)-1-propinyl]-benzoesäure

2-Acetoxy-4-[3-acetoxy-3-(5,6,7,8-tetrahydro-5,5,8,8,-tetramethyl-2-naphthyl)-1-propinyl]-benzoesäure

4-[3-Hydroxy-3-(3-*tert*.-butyl-4-propyloxyphenyl)-1-propinyl]-benzoesäuremethylester

4-[3-Hydroxy-3-(3-*tert*.-butyl-4-hexloxyphenyl)-1-propinyl]-benzoesäuremethylester

**16.** Verbindungen nach Anspruch 1, dadurch gekennzeichnet, daß $R_5$ -OH, $R_7$ eine Gruppe -$OR_8$ und $R_{11}$ eine Gruppe -$OR_6$ bedeuten, wobei $R_6$ und $R_8$ die oben angegebenen Bedeutungen aufweisen.

**17.** Verbindungen nach einem der vorhergehenden Ansprüche zur Verwendung als Arzneimittel.

**18.** Verbindungen nach Anspruch 17 zur Verwendung als Arzneimittel, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

**19.** Verwendung mindestens einer Verbindung nach den Ansprüchen 1 bis 16 zur Herstellung eines Arzneimittels, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

**20.** Pharmazeutische Zusammensetzung, dadurch gekennzeichnet, daß sie in einem pharmazeutisch akzeptablen Träger mindestens eine der Verbindungen nach einem der Ansprüche 1 bis 16 enthält.

**21.** Zusammensetzung nach Anspruch 20, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, liegt.

**22.** Kosmetische Zusammensetzung, dadurch gekennzeichnet, daß sie in einem kosmetisch akzeptablen Träger mindestens eine Verbindung nach einem der Ansprüche 1 bis 16 enthält.

**23.** Zusammensetzung nach Anspruch 22, dadurch gekennzeichnet, daß die Konzentration der Verbindung(en) nach einem der Ansprüche 1 bis 16 im Bereich von 0,001 bis 3 Gew.-%, bezogen auf die gesamte Zusammensetzung, liegt.

**24.** Verwendung einer kosmetischen Zusammensetzung nach einem der Ansprüche 22 oder 23 zur Körper- oder Haarhygiene.

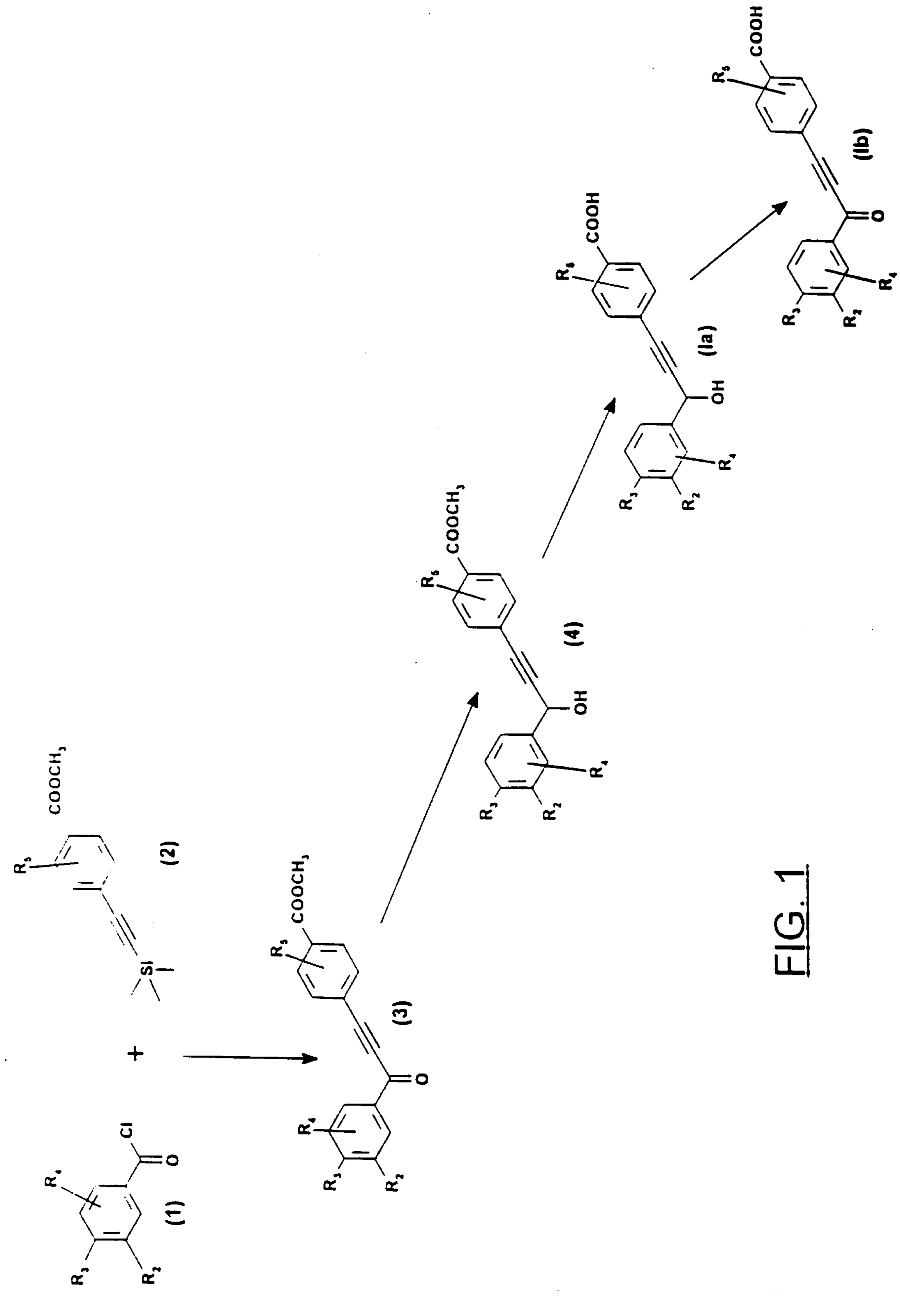

(1) (2) (3) (4) (Ia) (Ib)

FIG. 1

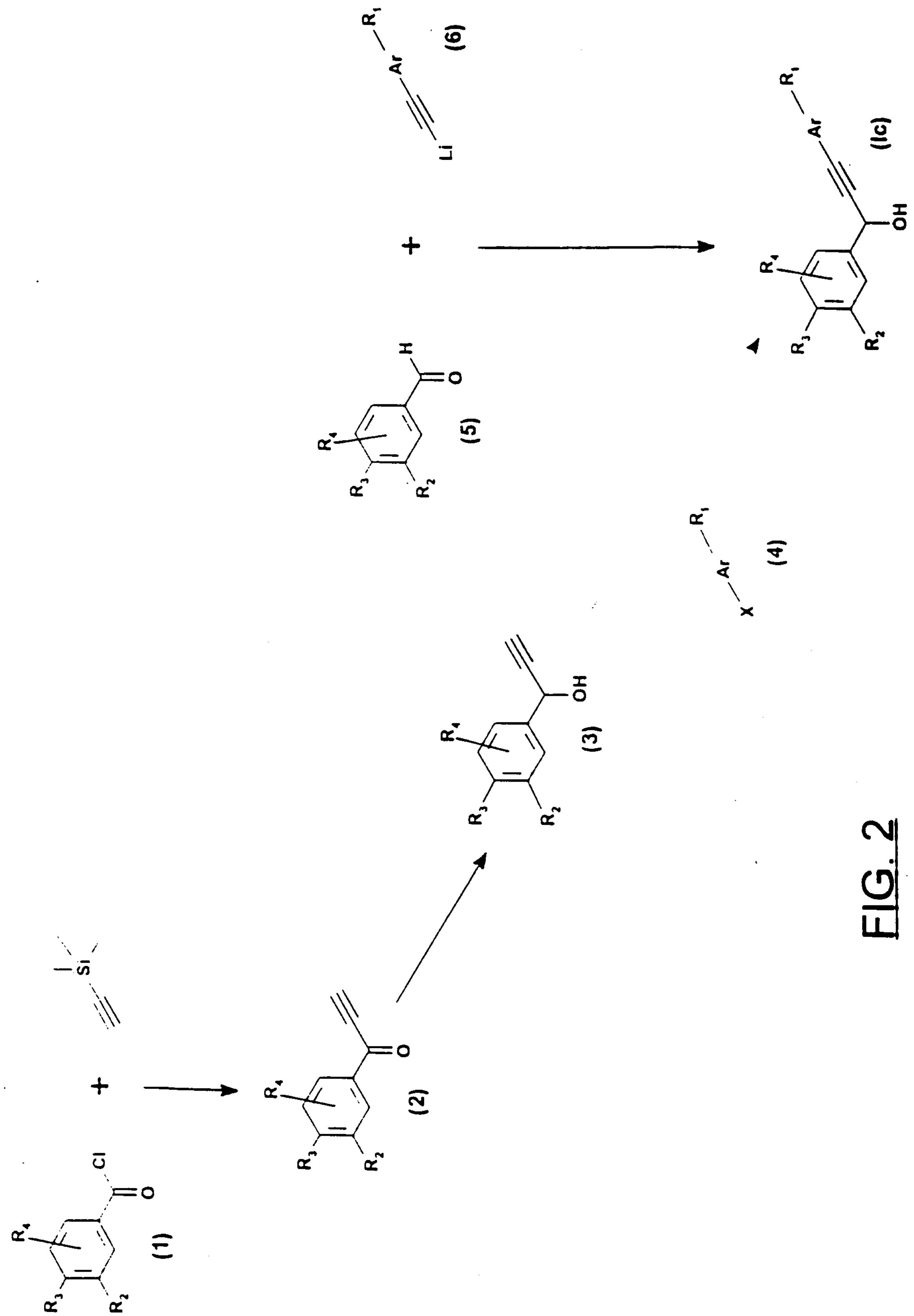

(1) (2) (3) (4) (5) (6) (Ic)

FIG. 2

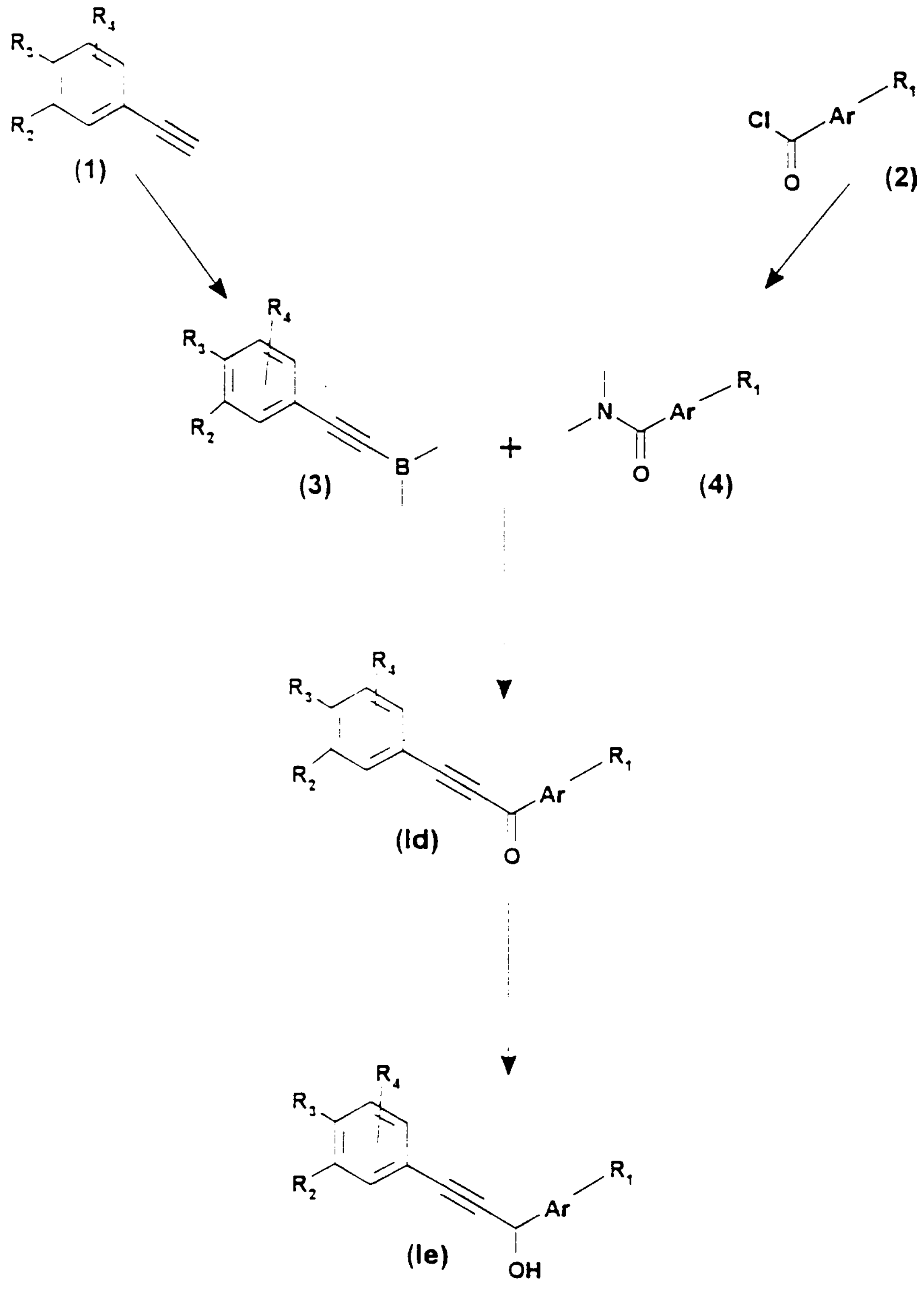

(1)

(2)

(3)

(4)

(Id)

(Ie)

FIG. 3